(19) Europäisches Patentamt / European Patent Office / Office européen des brevets

(11) **EP 4 282 323 B1**

(12) **EUROPEAN PATENT SPECIFICATION**

(45) Date of publication and mention
of the grant of the patent:
**02.07.2025  Bulletin 2025/27**

(21) Application number: **22847933.3**

(22) Date of filing: **06.05.2022**

(51) International Patent Classification (IPC):
*A61B 5/00* (2006.01)        *G06F 17/18* (2006.01)
*A61B 5/024* (2006.01)      *G16H 40/63* (2018.01)
*G16H 50/20* (2018.01)

(52) Cooperative Patent Classification (CPC):
**A61B 5/7221; A61B 5/02416; A61B 5/7207;
A61B 5/7257; G06F 17/18; G16H 40/63;
G16H 50/20;** G06F 2218/02; G06F 2218/08

(86) International application number:
**PCT/CN2022/091300**

(87) International publication number:
**WO 2023/005318 (02.02.2023 Gazette 2023/05)**

(54) **PHYSIOLOGICAL DETECTION SIGNAL QUALITY EVALUATION METHOD, ELECTRONIC DEVICE**

METHODE ZUR BEWERTUNG DER QUALITÄT VON PHYSIOLOGISCHEN ERKENNUNGSSIGNALEN, ELEKTRONISCHES GERÄT

MÉTHODE D'ÉVALUATION DE LA QUALITÉ DU SIGNAL DE DÉTECTION PHYSIOLOGIQUE, DISPOSITIF ÉLECTRONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.07.2021  CN 202110866931**

(43) Date of publication of application:
**29.11.2023  Bulletin 2023/48**

(73) Proprietor: **Beijing Honor Device Co., Ltd.
Beijing 100095 (CN)**

(72) Inventors:
• **ZHANG, Xiaowu
Shenzhen, Guangdong 518040 (CN)**
• **LI, Danhong
Shenzhen, Guangdong 518040 (CN)**

(74) Representative: **Beder, Jens
Mitscherlich PartmbB
Patent- und Rechtsanwälte
Karlstraße 7
80333 München (DE)**

(56) References cited:
CN-A- 109 475 311        US-A1- 2009 240 119
US-A1- 2019 133 533      US-A1- 2020 302 825
US-A1- 2020 337 574      US-A1- 2020 337 574

## Description

This application claims priority to Chinese Patent Application CN 115 700 538 A (Application No. 202110866931.X), filed with the China National Intellectual Property Administration on July 29, 2021 and entitled "PHYSIOLOGICAL DETECTION SIGNAL QUALITY EVALUATION METHOD, ELECTRONIC DEVICE, AND STORAGE MEDIUM".

## TECHNICAL FIELD

This application relates to the field of medical detection technologies, and in particular, to a physiological detection signal quality evaluation method and an electronic device.

## BACKGROUND

Nowadays, intelligent detection devices are used increasingly widely. A medical detection principle is mainly based on photoplethysmography (Photoplethysmography, PPG). PPG is a non-invasive detection method used to detect volumetric changes in blood vessel of a living tissue by using a photoelectric means. An original PPG signal usually includes a plurality of pieces of physiological information such as heart rate, oxygen saturation, and blood pressure. Because a detection device with a PPG measurement function is small in size, convenient to carry, and simple to use, the detection device is widely used for 24-hour monitoring of a plurality of physiological indices. However, such 24-hour monitoring is usually accompanied by a plurality of complex use scenarios, and the complex use scenario means that a PPG signal is susceptible to complex and changeable noise interference, and consequently, standard quantization cannot be performed on signal quality in a plurality of scenarios, and it is inconvenient to evaluate performance of hardware in the plurality of scenarios.

US 2020/337574 A1 discloses a method of collecting PPG data associated with a user using a biometric monitoring device, extracting a signal quality metric from the collected PPG data, classifying the PPG data signal into one of a plurality of signal quality levels based on the extracted signal quality metric, and determining whether to increase, decrease, or maintain a power level of an LED of the biometric monitoring device based on the classification.

## Summary of the invention

The invention is set out in the appended set of claims.

Summary of the disclosure

To solve the problem that standard quantization and evaluation cannot be performed on signal quality in a plurality of scenarios, embodiments of the present application provide a physiological detection signal quality evaluation method and an electronic device according to the enclosed independent claims. Advantageous features of the present invention are defined in the corresponding subclaims . In the following, parts of the description and drawings referring to embodiments, which are not covered by the claims, are not presented as embodiments of the invention but as examples useful for understanding the invention.

According to a first aspect, this application provides a physiological detection signal quality evaluation method. The method includes: obtaining a sample data set collected by a detection device in different scenarios; selecting sample data of a scenario from the sample data set, and dividing the sample data into physiological data and PPG data; calculating an energy ratio of the physiological data based on the physiological data and the PPG data; separately calculating quality evaluation parameters of sample data of a plurality of scenarios based on energy ratios of the physiological data corresponding to different scenarios; and evaluating, based on the quality evaluation parameters, quality of the physiological detection signal collected by the detection device. In the physiological detection signal quality evaluation method provided in this application, the energy ratio of the physiological data is calculated, to quantize quality of physiological detection signals collected by the device in different scenarios, so that it is convenient to evaluate and analyze signal quality.

In a possible implementation, the obtaining sample data collected by a detection device in different scenarios includes: setting a plurality of preset scenarios based on a label; connecting the detection device to a data collection end; collecting, by the detection device, data based on a single preset scenario to generate a single piece of sample data; and outputting a multi-scenario sample data set based on sample data of the plurality of preset scenarios. According to the foregoing technical solution, sample data in different scenarios can be collected conveniently and accurately.

The calculating an energy ratio of the physiological data based on the physiological data and the PPG data includes: separately calculating, based on the physiological data and the PPG data, a plurality of energy ratios of frequencies corresponding to a plurality of pieces of physiological data; and calculating an average value of the plurality of energy ratios of the frequencies corresponding to the plurality of pieces of physiological data, and using the average value as the energy ratio of the physiological data. In the foregoing technical solution, calculation precision of the energy ratio of the physiological data is improved by using a method for calculating an average value.

In a possible implementation, the separately calculating, based on the physiological data and the PPG data, a plurality of energy ratios of frequencies corresponding to a plurality of pieces of physiological data includes: performing short-time Fourier transform on the PPG data to obtain power spectral density data of the PPG data, where a window type of the short-time Fourier transform is a Hamming window; determining time points corresponding to center locations of a plurality of Hamming windows, and obtaining physiological data corresponding to each time point; converting the physiological data into a frequency range; calculating, based on the power spectral density data, an energy ratio corresponding to the frequency range; and outputting an energy ratio list of the plurality of pieces of physiological data in the sample data based on energy ratios corresponding to a plurality of frequency ranges. In the foregoing technical solution, calculation precision of the energy ratio of the physiological data is improved through frequency-domain analysis.

In a possible implementation, the calculating, based on the power spectral density data, an energy ratio corresponding to the frequency range includes: dividing a sum of energy values corresponding to a plurality of frequency values in the frequency range by a sum of energy values corresponding to all frequency values to obtain the energy ratio corresponding to the frequency range. In the foregoing technical solution, the energy ratio of the physiological data is calculated by using a power spectral density curve obtained through frequency-domain analysis, thereby improving calculation precision.

In a possible implementation, the separately calculating, based on the physiological data and the PPG data, a plurality of energy ratios of frequencies corresponding to a plurality of pieces of physiological data further includes: performing interpolation processing on the power spectral density data in a time-domain zero-filling or frequency-domain padding manner. In the foregoing technical solution, frequency-domain resolution may be increased, to improve calculation precision of the energy ratio.

In a possible implementation, the separately calculating, based on the physiological data and the PPG data, a plurality of energy ratios of frequencies corresponding to a plurality of pieces of physiological data further includes: deleting abnormal data in the energy ratio list of the plurality of pieces of physiological data. According to the foregoing technical solution, a calculation deviation of the energy ratio may be corrected, to improve validity of a sample.

In a possible implementation, the deleting abnormal data in the energy ratio list of the plurality of pieces of physiological data includes: calculating an upper quartile Q1E and a lower quartile Q3E in the energy ratio list; calculating an upper threshold U1 and a lower threshold U2 of the abnormal data; determining that data that is in the energy ratio list and that is less than or equal to the lower threshold or greater than or equal to the upper threshold is the abnormal data; and outputting an energy ratio list from which the abnormal data is deleted. In the foregoing technical solution, a boundary of a normal sample is determined by using a threshold, to accurately delete the abnormal data.

In a possible implementation, the upper threshold is $U_1 = a * Q_3^E - b * Q_1^E$, and the lower threshold is $U_2 = a * Q_1^E - b * Q_3^E$. In the foregoing technical solution, a threshold is determined by using a preset and adjustable weight or coefficient, so that the threshold is adjustable and is suitable for different application scenarios.

In a possible implementation, the quality evaluation parameters include a lower quartile, a median, and an upper quartile of physiological data energy ratios of a plurality of scenarios in the classified scenarios. In the foregoing technical solution, signal quality is quantized by using statistical data, so that the quantized data can accurately reflect a feature of original data.

In a possible implementation, the separately calculating quality evaluation parameters of sample data of a plurality of classified scenarios based on energy ratios of the physiological data corresponding to different scenarios includes: classifying the different scenarios to determine the plurality of classified scenarios; obtaining, based on an analysis object, a plurality of physiological data energy ratios of classified scenarios separately corresponding to at least two evaluation dimensions; and calculating a lower quartile, a median, and an upper quartile of the physiological data energy ratios of classified scenarios separately corresponding to the at least two evaluation dimensions. In the foregoing technical solution, signal quality is evaluated based on different analysis objects, different evaluation dimensions, and quality evaluation parameters of different classified scenarios, so that evaluation accuracy is improved.

In a possible implementation, the evaluating, based on the quality evaluation parameters, quality of a physiological detection signal collected by the detection device includes: determining, based on an increase or decrease ratio of a quality evaluation parameter of a classified scenario corresponding to a first evaluation dimension to a quality evaluation parameter of a classified scenario corresponding to a second evaluation dimension, whether the quality of the physiological detection signal collected by the detection device is improved or degraded. In the foregoing technical solution, improvement or degradation of quality can be intuitively reflected by the increase or decrease ratio.

In a possible implementation, the determining, based on an increase or decrease ratio of a quality evaluation parameter of a classified scenario corresponding to a first evaluation dimension to a quality evaluation parameter of a classified scenario corresponding to a second evaluation dimension, whether the quality of the physiological detection signal collected by the detection device is improved or degraded includes: calculating a lower quartile increase ratio, a median increase ratio, and an upper quartile increase ratio of a plurality of physiological data energy ratios of the classified scenario corresponding to the first evaluation dimension to a plurality of physiological data energy ratios of the classified scenario corresponding to the second evaluation dimension; and determining, based on the lower quartile increase ratio, the median increase ratio, and the upper quartile increase ratio, whether the quality of the physiological detection signal collected by the detection device is improved or degraded. In the foregoing technical solution, improvement or degradation of quality can be intuitively reflected by an increase or decrease of statistical data.

In a possible implementation, the determining, based on the lower quartile increase ratio, the median increase ratio, and the upper quartile increase ratio, whether the quality of the physiological detection signal collected by the detection device is improved or degraded includes: determining whether the lower quartile increase ratio, the median increase ratio, and the upper quartile increase ratio are greater than 0; and if it is determined that the lower quartile increase ratio, the median increase ratio, and the upper quartile increase ratio are greater than 0, determining that quality of a physiological detection signal collected by the detection device in the first evaluation dimension is improved relative to that in the second evaluation dimension; if it is determined that the lower quartile increase ratio, the median increase ratio, and the upper quartile increase ratio are all equal to 0, determining that quality of a physiological detection signal collected by the detection device in the first evaluation dimension remains unchanged relative to that in the second evaluation dimension; or if it is determined that any one of the lower quartile increase ratio, the median increase ratio, and the upper quartile increase ratio is less than 0, determining that quality of a physiological detection signal collected by the detection device in the first evaluation dimension is degraded relative to that in the second evaluation dimension.

In a possible implementation, the determining, based on the lower quartile increase ratio, the median increase ratio, and the upper quartile increase ratio, whether the quality of the physiological detection signal collected by the detection device is improved or degraded includes: calculating an average value of the lower quartile increase ratio, the median increase ratio, and the upper quartile increase ratio; determining whether the average value is greater than 0; and if it is determined that the average value is greater than 0, determining that quality of a physiological detection signal collected by the detection device in the first evaluation dimension is improved relative to that in the second evaluation dimension; if it is determined that the average value is equal to 0, determining that quality of a physiological detection signal collected by the detection device in the first evaluation dimension remains unchanged relative to that in the second evaluation dimension; or if it is determined that the average value is less than 0, determining that quality of a physiological detection signal collected by the detection device in the first evaluation dimension is degraded relative to that in the second evaluation dimension. According to the foregoing technical solution, whether the quality of the physiological detection signal is improved can be accurately determined.

In a possible implementation, the determining, based on the lower quartile increase ratio, the median increase ratio, and the upper quartile increase ratio, whether the quality of the physiological detection signal collected by the detection device is improved or degraded includes: separately setting weight values of the lower quartile increase ratio, the median increase ratio, and the upper quartile increase ratio; calculating a sum of the lower quartile increase ratio, the median increase ratio, and the upper quartile increase ratio based on the weight values of the lower quartile increase ratio, the median increase ratio, and the upper quartile increase ratio; determining whether the sum of the lower quartile increase ratio, the median increase ratio, and the upper quartile increase ratio is greater than 0; and if it is determined that the sum of the lower quartile increase ratio, the median increase ratio, and the upper quartile increase ratio is greater than 0, determining that quality of a physiological detection signal collected by the detection device in the first evaluation dimension is improved relative to that in the second evaluation dimension; if it is determined that the sum of the lower quartile increase ratio, the median increase ratio, and the upper quartile increase ratio is equal to 0, determining that quality of a physiological detection signal collected by the detection device in the first evaluation dimension remains unchanged relative to that in the second evaluation dimension; or if it is determined that the sum of the lower quartile increase ratio, the median increase ratio, and the upper quartile increase ratio is less than 0, determining that quality of a physiological detection signal collected by the detection device in the first evaluation dimension is degraded relative to that in the second evaluation dimension. In the foregoing technical solution, through setting of weights, adjustment space of a signal quality evaluation manner can be improved, and importance of different quality evaluation parameters can be reflected.

In a possible implementation, the method further includes: performing upsampling processing on the PPG data in the sample data. In the foregoing technical solution, resolution of sample data is increased to improve precision of signal quality evaluation.

In a possible implementation, the method further includes: filtering out low-frequency and/or high-frequency noise in the PPG data in the sample data. In the foregoing technical solution, interference caused by the low-frequency and/or high-frequency noise to signal quality evaluation can be eliminated, so that precision of signal quality evaluation is improved.

In a possible implementation, the filtering out low-frequency and/or high-frequency noise in the PPG data in the sample data includes: setting an upper threshold and a lower threshold of a band-pass frequency by using an f-order band-pass filter; and inputting the PPG data into the band-pass filter, and filtering out the low-frequency and/or high-frequency noise in the PPG data based on the upper threshold and the lower threshold by using the band-pass filter. According to the foregoing technical solution, the low-frequency and/or high-frequency noise can be accurately filtered out.

According to a second aspect, this application provides an electronic device. The electronic device includes a memory and a processor.

The memory is configured to store program instructions.

The processor is configured to read and execute the program instructions stored in the memory, and when the program instructions are executed by the processor, the electronic device is enabled to perform the foregoing physiological detection signal quality evaluation method.

According to a third aspect, this application provides a computer storage medium. The computer storage medium stores program instructions, and when the program instructions are run on an electronic device, the electronic device is enabled to perform the foregoing physiological detection signal quality evaluation method.

In addition, for technical effects brought by the second aspect and the third aspect, refer to the related descriptions of the method in the designs of the method part. Details are not described herein again.

According to the physiological detection signal quality evaluation method, the electronic device, and the storage medium that are provided in this application, quality of a physiological detection signal may be quantized, and it is convenient to evaluate the quality of the physiological detection signal based on quantized data, to well adapt to different use scenarios, thereby facilitating evaluation of multi-scenario performance of hardware.

BRIEF DESCRIPTION OF DRAWINGS

FIG. 1A is a schematic diagram showing that a user wears a detection device in a sports scenario according to an embodiment of this application;
FIG. 1B is a schematic diagram showing that a user wears a detection device in a stationary scenario according to an embodiment of this application;
FIG. 1C is a schematic diagram showing that a user wears a detection device in a sleeping scenario according to an embodiment of this application;
FIG. 2 is a schematic diagram of a communication architecture of an electronic device according to an embodiment of this application;
FIG. 3 is a flowchart of a physiological detection signal quality evaluation method according to an embodiment of this application;
FIG. 4 is a flowchart of obtaining a sample data set of a detection device in different scenarios according to an embodiment of this application;
FIG. 5 is a flowchart of calculating an energy ratio of physiological data in sample data according to an embodiment of this application;
FIG. 6A is a schematic diagram of a power spectral density curve before interpolation processing according to an embodiment of this application;
FIG. 6B is a schematic diagram of a power spectral density curve after interpolation processing according to an embodiment of this application;
FIG. 7 is a schematic diagram of a power spectral density curve according to an embodiment of this application;
FIG. 8 is a flowchart of deleting abnormal data according to an embodiment of this application;
FIG. 9 is a box-shaped diagram of energy ratios of a plurality of pieces of physiological data in sample data of different scenarios;
FIG. 10 is a flowchart of calculating a quality evaluation parameter according to an embodiment of this application;
FIG. 11 is a flowchart of a physiological detection signal quality evaluation method according to another embodiment of this application;
FIG. 12 is a flowchart of a physiological detection signal quality evaluation method according to another embodiment of this application;
FIG. 13 is a flowchart of a physiological detection signal quality evaluation method according to another embodiment of this application; and
FIG. 14 is a diagram of an architecture of an electronic device according to an embodiment of this application.

DESCRIPTION OF EMBODIMENTS

The following terms "first" and "second" are used merely for the purpose of description, and shall not be construed as indicating or implying relative importance or implying a quantity of indicated technical features. Therefore, a feature defined by "first" or "second" may explicitly or implicitly include one or more such features. In the descriptions of embodiments of this application, words such as "for example" or "such as" are used to mean an example, an illustration, or a description. Any embodiment or design solution described as "example" or "for example" in embodiments of this application should not be construed as being more preferred or advantageous than other embodiments or design solutions. Exactly, use of the word such as "as an example" or "for example" is intended to present related concepts in a specific manner.

Unless otherwise specified, all technical and scientific terms used in this specification have meanings that are the same as those commonly understood by a person skilled in the art of this application. The terms used in the specification of this application are merely intended to describe specific embodiments, but are not intended to limit this application. It should be understood that, in this application, unless otherwise stated, "/" indicates "or". For example, A/B may indicate A or B. In this application, "and/or" is merely an association relationship that describes associated objects, and indicates that three relationships may exist. For example, A and/or B may indicate the following three cases: Only A exists, both A and B exist, and only B exists. "At least one" means one or more. "A plurality of" means two or more. For example, at least one of a, b, or c may indicate seven cases: a, b, c, a and b, a and c, b and c, and a, b, and c.

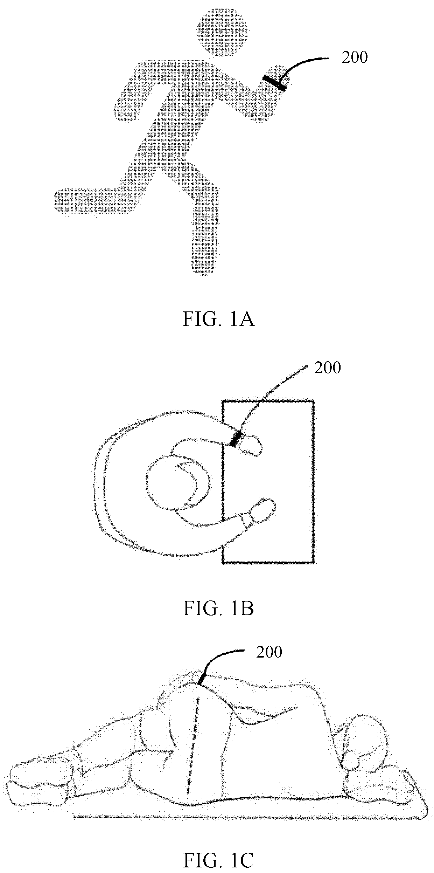

FIG. 1A - FIG. 1C are schematic diagrams in which a detection device is applied to different scenarios. Actual user experience is used as an example. A user is in an outdoor sports state, an indoor sitting state, a sleeping state, and the like all day long. In different states, the user may use the detection device to detect a physiological parameter. In a detection process of the physiological parameter, PPG is a main detection means. PPG is a non-invasive detection method used to detect volumetric changes in blood vessel of a living tissue by using a photoelectric means. An original PPG signal includes a plurality of pieces of physiological information such as heart rate, oxygen saturation, and blood pressure. Because a PPG measurement device is small in size, convenient to carry, and simple to use, the PPG measurement device is widely used for 24-hour monitoring of a plurality of physiological indices. However, such 24-hour monitoring is usually accompanied by a plurality of complex use scenarios, and the complex use scenario means that the PPG signal is susceptible to complex and changeable noise interference. Because an antiinterference capability of a hardware device is strongly related to validity of an algorithm developed based on a PPG signal generated by the device, standard quantization cannot be accurately performed on signal quality in a plurality of scenarios, it is inconvenient to reasonably evaluate performance of hardware in the plurality of scenarios, and it is inconvenient to optimize a specific scenario of the hardware.

In addition, currently, an evaluation indicator used by a hardware developer is applicable to a single scenario. Although many hardware vendors have a related evaluation solution, the solution is not detailed. In addition, an evaluation object at which the indicator is targeted is a single sample rather than a multi-scenario sample set, and there is no capability of discriminating and correcting a deviation caused by a gold standard.

To resolve a technical problem that PPG signal quality of the detection device cannot be effectively evaluated, an embodiment of this application provides a physiological detection signal quality evaluation method. The method is applied to an electronic device 100.

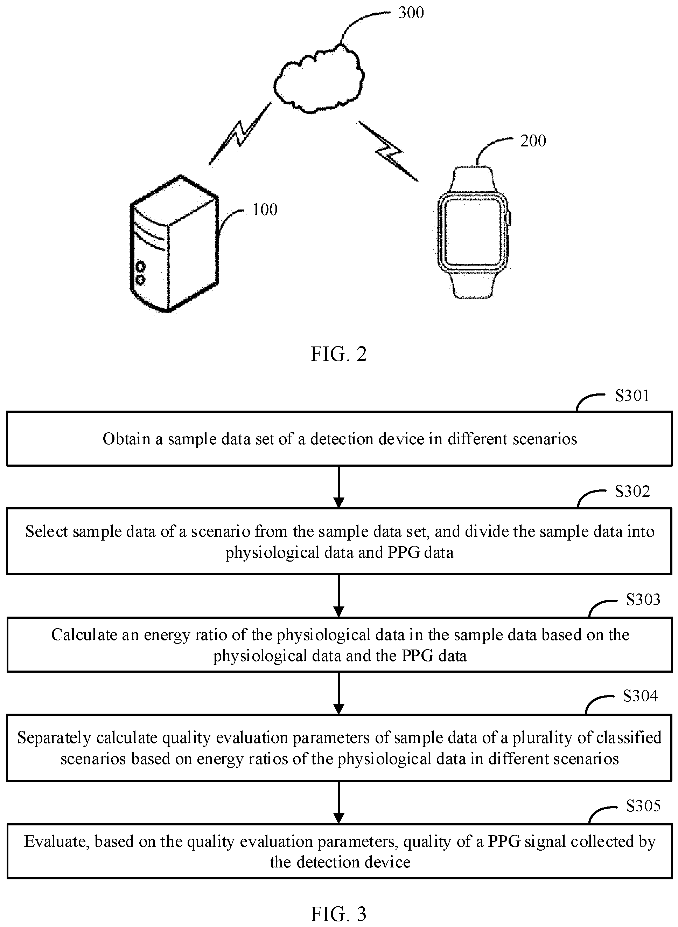

FIG. 2 is a schematic diagram of a communication architecture of the electronic device according to an embodiment of this application. The electronic device 100 is communicatively connected to a detection device 200 by using a network 300. For example, the network 300 may be at least one of a 2G network, a 3G network, a 4G-LET network, or a 5G new radio (5G-New Radio) network. It should be noted that the foregoing description does not constitute a limitation on the communication architecture of the electronic device 100 in this embodiment of this application. A communication architecture diagram of the electronic device 100 in this embodiment of this application is not limited to the example shown in FIG. 2. The detection device 200 may be a device for physiological detection, for example, a cardiotachometer or a pulsimeter, or may be a device with a physiological detection function, for example, an electronic wristband or a smartwatch.

FIG. 3 is a flowchart of a physiological detection signal quality evaluation method according to an embodiment of this application. The method is applied to an electronic device 100, and specifically includes the following steps.

S301: Obtain a sample data set of a detection device in different scenarios.

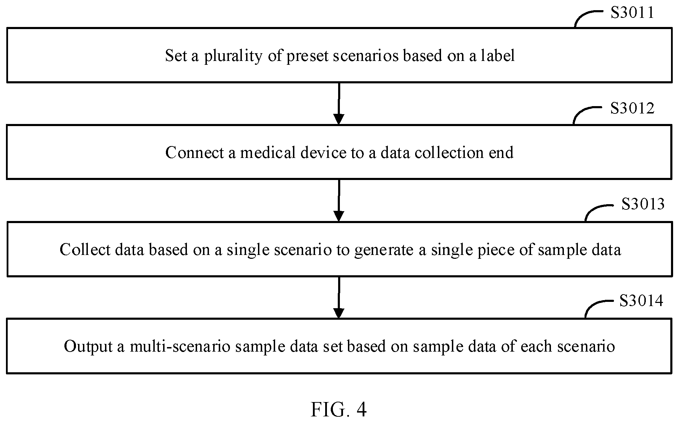

In an embodiment of this application, for the obtaining of sample data of the detection device in the different scenarios, refer to a detailed procedure in FIG. 4. The obtaining of the sample data of the detection device in the different scenarios specifically includes: S3011: Set a plurality of preset scenarios based on a label. The label may be set based on a requirement, and may include but is not limited to a user status, an environment in which a user is located, and a sports type. For example, if the label is all, the plurality of preset scenarios may include all scenarios to which the detection device may be applied; if the label is indoor, the plurality of preset scenarios may include indoor sports, indoor stationariness, and the like; if the label is outdoor, the plurality of preset scenarios may include outdoor sports, outdoor stationariness, and the like; if the label is stationary, the plurality of preset scenarios may include indoor stationariness, outdoor stationariness, and the like; if the label is sports, the plurality of preset scenarios may include indoor sports, outdoor sports, and the like; if the label is sports type, the plurality of preset scenarios may include cycling, walking, running, and the like; and if the label is state, the plurality of preset scenarios may include stationariness, sports, and the like.

In an embodiment of this application, the preset scenarios may be prepared by using a simulation device. For example, the simulation device may be a robot, and the detection device is disposed on the robot, to implement the plurality of preset scenarios. In another embodiment, the preset scenarios may alternatively be prepared by using an actual user and an actual environment, and the detection device is worn by the user, and is separately located in different environments, to implement the plurality of preset scenarios.

S3012: Connect the detection device to a data collection end. Specifically, accessories of the detection device are separately connected to the data collection end. For example, the detection device is an electronic wristband with a heart rate collection function, and the electronic wristband includes a wristband and a heart rate belt. The wristband is worn on the wrist of the user, and a PPG signal of the user may be collected in real time by a sensor. The heart rate belt is worn near the heart, and an electrocardiogram signal of the user may be collected in real time by the sensor.

In an embodiment of this application, the data collection end is an application installed and running on the detection device, and the application is used to collect data collected by the sensor. The detection device may send the PPG signal and the electrocardiogram signal that are collected by the sensor to the application in real time, and transmit the PPG signal and the electrocardiogram signal to the electronic device through a network.

In other embodiments, the data collection end is an application installed and running on the electronic device, and the application is used to collect data collected by the detection device. The detection device may send the PPG electronic device and the electrocardiogram signal that are collected by the sensor to the electronic device through a network in real time, and the electronic device stores the received PPG signal and electrocardiogram signal in the application.

S3013: Collect data based on a single scenario to generate a single piece of sample data. Specifically, in any one of the plurality of scenarios, the user performs an action based on the scenario. For example, if the scenario is outdoor sports, the user does sports in the outdoor. In a process in which the user performs the action, the detection device starts to work, collects a preset quantity of PPG signals and electrocardiogram signals by using the sensor, and stops collecting after an amount of collected sample data reaches a standard. Optionally, the preset quantity is 50, that is, 50 PPG signals and 50 electrocardiogram signals are separately collected.

S3014: Output a multi-scenario sample data set based on sample data of each scenario. In an embodiment of this application, sample data in all scenarios is summarized to output the multi-scenario sample data set.

S302: Select sample data of a scenario from the sample data set, and divide the sample data into physiological data and PPG data.

In an embodiment of this application, the selecting sample data of a scenario from the sample data set includes: randomly selecting sample data of a scenario from the output multi-scenario sample data set.

In an embodiment of this application, the dividing the sample data into physiological data and PPG data includes: converting a PPG signal in the sample data into physiological data H, and converting the PPG signal into single-channel PPG data $P_0$. Preferably, the physiological data H is heart rate label data, and the PPG data is detected and obtained by a PPG sensor of the detection device. The PPG sensor converts a transmitted optical signal and an optical signal that is reflected by a human body tissue (muscle, bone, vein, or the like) into electrical signals, to obtain the PPG data. The electrical signal may be a voltage signal. In other words, the PPG data is a voltage. In other embodiments, the physiological data H may alternatively be pulse data.

S303: Calculate an energy ratio of the physiological data in the sample data based on the physiological data H and the PPG data $P_0$.

In an embodiment of this application, a plurality of energy ratios of frequencies corresponding to a plurality of pieces of physiological data are separately calculated based on the physiological data H and the PPG data $P_0$, and an average value of the plurality of energy ratios of the frequencies corresponding to the plurality of pieces of physiological data is calculated and used as the energy ratio of the physiological data.

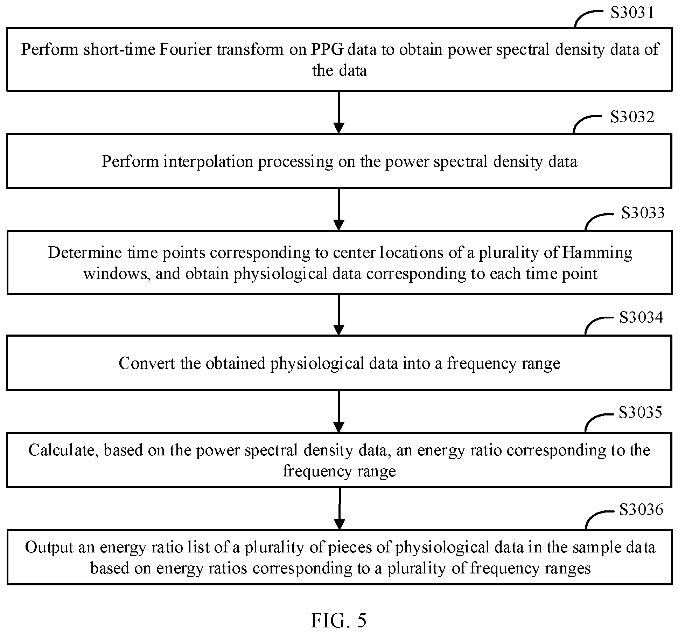

Specifically, as shown in a detailed procedure in FIG. 5, in an embodiment of this application, the separately calculating, based on the physiological data H and the PPG data $P_0$, a plurality of energy ratios of frequencies corresponding to a plurality of pieces of physiological data includes: S303 1: Perform short-time Fourier transform on the PPG data $P_0$ to obtain power spectral density data of the PPG data $P_0$. Because the preset quantity of PPG signals are collected, the preset quantity of PPG signals may be formed through conversion. The physiological data is a heart rate value per second. An expression of the short-time Fourier transform is:

$$STFT_Z(t,f) = \int_{-\infty}^{\infty} [z(u)g(u-t)]e^{-j2\pi fu}du.$$

z(u) is an original PPG signal, and g(u-t) is a window function. A window type of the short-time Fourier transform is a Hamming window, a window length is a data length of 2t (where t is an integer) seconds, and a moving step is a data length of one second. It is assumed that the preset amount of PPG data is $P_0 = [(t_0, x_0), (t_1, x_1), ..., (t_n, x_n)]$, and power spectral density data $Q = [(f_1, p_t), (f_2, p_{t+k}), ... (f_i, p_g)]$ is obtained after the short-time Fourier transform is performed on the PPG data $P_0$, and is used as a frequency-domain feature of the PPG data $P_0$.

S3032: Perform interpolation processing on the power spectral density data.

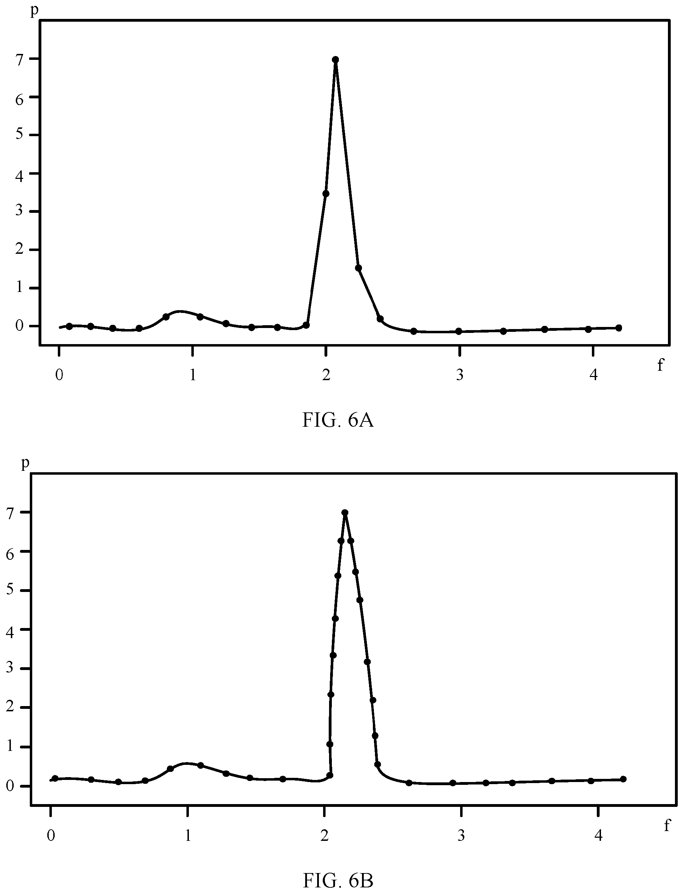

FIG. 6A shows an original power spectral density curve. In an embodiment of this application, the interpolation processing is performed on the power spectral density data in a frequency-domain padding manner. Specifically, discrete power spectral density data is fitted into a quadratic spline curve, and interpolation processing of a first preset multiple is performed based on the quadratic spline curve. FIG. 6B shows a power spectral density curve obtained after the interpolation processing. An expression of the quadratic spline curve is:

$$P(t) = A_1 + A_2 t + A_3 t^2 \ (0 \le t \le 1).$$

$A_1$, $A_2$, and $A_3$ are coefficients in a two-dimensional vector form. Optionally, the first preset multiple is 50 times. The power spectral density data can be expanded to 50 times the original power spectral density data through the interpolation processing, to improve precision of subsequent analysis of PPG signal quality. For example, if initial power spectral density data includes 100 pieces of data, power spectral density data obtained after the interpolation processing includes 5000 pieces of data.

In another embodiment of this application, the interpolation processing may alternatively be performed on the power spectral density data in a time-domain zero-filling manner. Specifically, before the short-time Fourier transform is performed on the PPG data, interpolation processing of a first preset multiple is performed on the PPG data in a time-domain zero-filling manner, and then the short-time Fourier transform is performed on PPG data obtained after the interpolation processing, to obtain power spectral density data that is 50 times the original data. For example, if an initial PPG signal includes 100 pieces of data, 4900 zeros are filled into the PPG signal.

S3033: Determine time points z corresponding to center locations of a plurality of Hamming windows, and obtain physiological data (for example, a heart rate value) corresponding to each time point z.

For example, if a time point corresponding to a center location of a Hamming window is a fifth second, a heart rate value at the fifth second is obtained from the physiological data H. A plurality of heart rate values can be obtained by using the plurality of Hamming windows.

S3034: Convert the obtained physiological data into a frequency range.

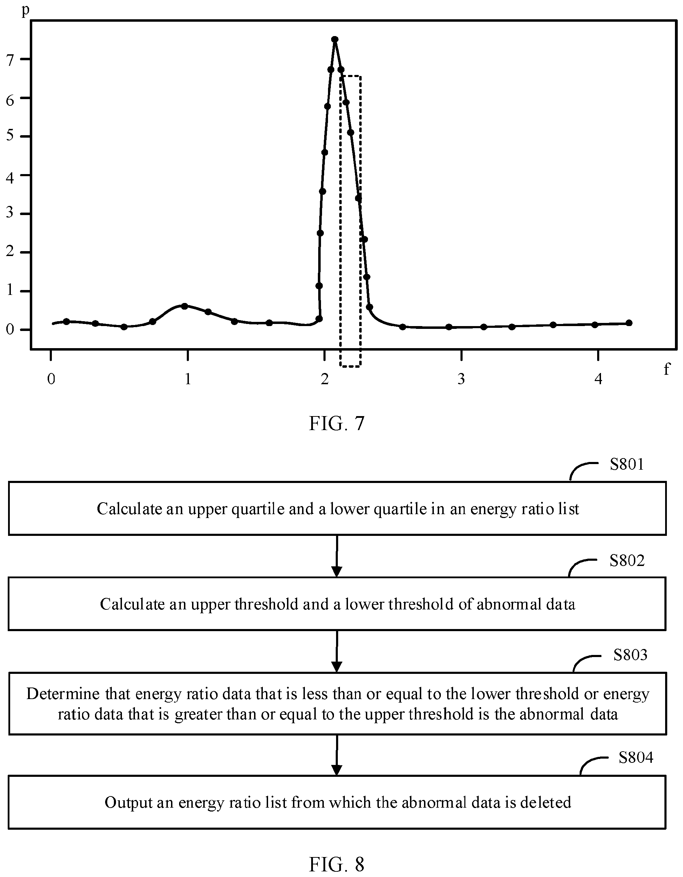

FIG. 7 shows a power spectral density curve obtained after the interpolation processing. The power spectral density curve is generated based on the power spectral density data, where a horizontal axis is frequency, and a vertical axis is energy. In an embodiment of this application, physiological data corresponding to the time point z is a heart rate value per minute, for example, 80 beats per minute (BPM), and the heart rate value is converted into a heart rate per second, that is, 1.33 times/second, to correspond to a frequency on the horizontal axis of the power spectral density curve. Then, a corresponding frequency range is determined based on a preset range of the physiological data. Optionally, a heart rate is used as an example, and the preset range is a range of $\pm 5$ BPM. For example, if the heart rate value is 80 BPM, a range of the heart rate value is 75-85 BPM, and a frequency range corresponding to the range of the heart rate value is 1.25-1.42 times/second (Hz).

S3035: Calculate, based on the power spectral density data, an energy ratio corresponding to the frequency range.

In an embodiment of this application, a formula for calculating the energy ratio IER corresponding to the frequency range is:

$$IER = \frac{signal\ power}{signal\ power + noise\ power},$$

where

signal power is an energy value in the frequency range, and noise power is an energy value outside the frequency range. Based on the foregoing example, a vertical axis value of a frequency (that is, each frequency value) corresponding to 1.25-1.42 Hz in the power spectral density curve is determined, that is, an energy value, signal power is a sum of energy values of corresponding frequencies within a range of 1.25-1.42 Hz, and noise power is a sum of energy values of all other frequencies.

S3036: Output an energy ratio list of the plurality of pieces of physiological data in the sample data based on energy ratios corresponding to a plurality of frequency ranges.

In an embodiment of this application, energy ratios of all physiological data is summarized to form the energy ratio list, and the energy ratio list is output. For example, the energy ratio list is $E = [e_1, e_2, \ldots, e_n]$.

In an embodiment of this application, a formula for calculating the average value of the plurality of energy ratios of the frequencies corresponding to the plurality of pieces of physiological data is:

$$R = mean(E).$$

In other words, an average value of all energy ratios in the energy ratio list is calculated, to obtain an energy ratio R of the physiological data in the sample data.

In an embodiment of this application, if an energy ratio R of physiological data in sample data corresponding to a current scenario is calculated, an energy ratio R of physiological data in a single piece of sample data corresponding to another scenario is calculated by using the foregoing method, until energy ratios R of physiological data in all single sample data are calculated.

In another embodiment of this application, the separately calculating, based on the physiological data H and the PPG data $P_0$, a plurality of energy ratios of frequencies corresponding to the plurality of pieces of physiological data further includes: deleting abnormal data in the energy ratio list E of the plurality of pieces of physiological data in the sample data.

Specifically, as shown in a detailed procedure in FIG. 8, in an embodiment of this application, the deleting abnormal data in the energy ratio list E of the plurality of pieces of physiological data in the sample data includes: S801: Calculate an upper quartile $Q_1^E$ and a lower quartile $Q_3^E$ in the energy ratio list E. A location of the upper quartile $Q_1^E$ is equal to (n+1)*0.75, a location of the lower quartile $Q_3^E$ is equal to (n+1)*0.25, where n is a quantity of frames (that is, a total amount of energy ratio data) in the energy ratio list E.

S802: Calculate an upper threshold and a lower threshold of the abnormal data.

In an embodiment of this application, the upper threshold of the abnormal data is $U_1 = a * Q_3^E - b * Q_1^E$, and the lower threshold of the abnormal data is $U_2 = a * Q_1^E - b * Q_3^E$. Values of a and b may be preset and adjusted. Optionally, a is 2.5, and b is 1.5.

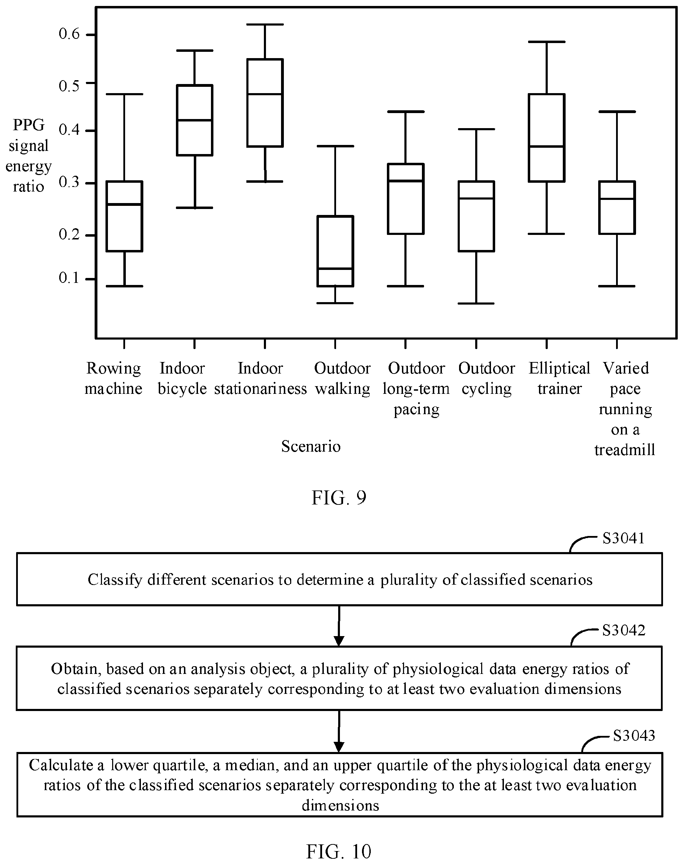

FIG. 9 is a box-shaped diagram of energy ratios of physiological data in sample data of different scenarios. The box-shaped diagram is generated based on the upper quartile $Q_1^E$ and the lower quartile $Q_3^E$ in the energy ratio list E, the upper threshold $U_1$, and the lower threshold $U_2$.

S803: Determine that energy ratio data that is less than or equal to the lower threshold $U_2$ or energy ratio data that is greater than or equal to the upper threshold $U_1$ is the abnormal data.

As shown in FIG. 9, an uppermost line of each box shape corresponds to the upper threshold $U_1$, a lowermost line corresponds to the lower threshold $U_2$, and a line inside a rectangle corresponds to a median of a plurality of energy ratios. The energy ratio data that is less than or equal to the lower threshold $U_2$ or the energy ratio data that is greater than or equal to the upper threshold $U_1$ is located outside the box-shaped diagram of the energy ratios, and therefore is the abnormal data.

S804: Output an energy ratio list $E_p$ from which the abnormal data is deleted.

In an embodiment of this application, after the abnormal data in the energy ratio list E is deleted, the energy ratio list $E_p$ is obtained, and the energy ratio list $E_p$ is output.

In another embodiment of this application, a formula for calculating the average value of the plurality of energy ratios of the frequencies corresponding to the plurality of pieces of physiological data is:

$$R = mean(E_p).$$

S304: Separately calculate quality evaluation parameters of sample data of a plurality of classified scenarios based on energy ratios of the physiological data in different scenarios.

In an embodiment of this application, the quality evaluation parameters include but are not limited to a lower quartile $Q_1$, a median $Q_2$, and an upper quartile $Q_3$ of the energy ratio. A location of the lower quartile $Q_1$ is equal to (n+1)*0.25, a location of the median $Q_2$ is equal to (n+1)*0.5, and a location of the upper quartile $Q_3$ is equal to (n+1)*0.75.

Specifically, as shown in a detailed procedure in FIG. 10, the separately calculating quality evaluation parameters of sample data of a plurality of classified scenarios based on energy ratios of the physiological data in different scenarios includes: S3041: Classify the different scenarios to determine the plurality of classified scenarios. In an embodiment of this application, a plurality of different scenarios may be classified based on a label, and a plurality of scenarios with a same label are classified into one classified scenario. For example, if the label is indoor, indoor sports, indoor stationariness, and the like may be classified into an indoor scenario; if the label is outdoor, outdoor sports, outdoor stationariness, and the like may be classified into an outdoor scenario; if the label is stationary, indoor stationariness, outdoor stationariness, and the like may be included; if the label is sports, the plurality of preset scenarios may include indoor sports, outdoor sports, and the like; and if the label is cycling, cycling scenarios of different users may be classified into a cycling scenario.

S3042: Obtain, based on an analysis object, a plurality of physiological data energy ratios of classified scenarios separately corresponding to at least two evaluation dimensions.

In an embodiment of this application, the analysis object may be set based on a requirement from different dimensions such as a device dimension, a scenario dimension, or a user dimension. For example, an analysis object corresponding to the device dimension may be: evaluating quality of PPG signals collected by detection devices of a same type but different versions, and evaluating quality of PPG signals collected by detection devices of different types. An analysis object corresponding to the scenario dimension may be: evaluating quality of PPG signals in different classified scenarios. An analysis object corresponding to the user dimension may be: evaluating quality of PPG signals collected in a same classified scenario when a same detection device is worn on different body parts of the user, and evaluating quality of PPG signals collected in a same classified scenario when a same detection device detects users of different age groups.

In an embodiment of this application, the evaluation dimension is determined based on the analysis object, and is at least two reference objects for an analysis object. For example, the at least two evaluation dimensions may include a previous-generation detection device and a new-generation detection device, and may include an electronic wristband and a smartwatch, or may include an age group of 20-30 years old and an age group of 30-40 years old.

After the evaluation dimension is determined, the plurality of physiological data energy ratios of the classified scenarios are obtained. For example, a first evaluation dimension is the previous-generation detection device, a second evaluation dimension is the new-generation detection device, and the classified scenario is a stationary scenario. A plurality of physiological data energy ratios of the previous-generation detection device in the stationary scenario (for example, indoor stationariness or outdoor stationariness) and a plurality of physiological data energy ratios of the new-generation detection device in the stationary scenario (for example, indoor stationariness or outdoor stationariness) are obtained.

S3043: Calculate a lower quartile, a median, and an upper quartile of the physiological data energy ratios of the classified scenarios separately corresponding to the at least two evaluation dimensions.

Based on the foregoing example, a lower quartile, a median, and an upper quartile of the energy ratios of the plurality of physiological data energy ratios of the previous-generation detection device in the stationary scenario and a lower quartile, a median, and an upper quartile of the energy ratios of the plurality of physiological data energy ratios of the new-generation detection device in the stationary scenario are calculated.

S305: Evaluate, based on the quality evaluation parameters, quality of a PPG signal collected by the detection device.

In an embodiment of this application, it is determined, based on an increase/decrease ratio of a quality evaluation parameter of a classified scenario corresponding to the first evaluation dimension to a quality evaluation parameter of a classified scenario corresponding to the second evaluation dimension, whether the quality of the PPG signal collected by the detection device is improved or degraded.

Specifically, if a first increase ratio of a lower quartile of a plurality of physiological data energy ratios in the first evaluation dimension in the classified scenario to a lower quartile of a plurality of physiological data energy ratios in the second evaluation dimension in the classified scenario, a second increase ratio of a median of the plurality of physiological data energy ratios in the first evaluation dimension in the classified scenario to a median of the plurality of physiological data energy ratios in the second evaluation dimension in the classified scenario, and a third increase ratio of an upper quartile of the plurality of physiological data energy ratios in the first evaluation dimension in the classified scenario to an upper quartile of the plurality of physiological data energy ratios in the second evaluation dimension in the classified scenario are all greater than 0, it is determined that quality of the PPG signal in the first evaluation dimension is improved relative to that in the second evaluation dimension. If the first increase ratio, the second increase ratio, and the third increase ratio are all equal to 0, it is determined that the quality of the PPG signal in the first evaluation dimension remains unchanged relative to that in the second evaluation dimension. If any one of the first increase ratio, the second increase ratio, or the third increase ratio is less than 0, it is determined that the quality of the PPG signal in the first evaluation dimension is degraded relative to that in the second evaluation dimension.

For example, a lower quartile of an energy ratio of the new-generation detection device in the stationary scenario is $Q_1^{new}$, a median is $Q_2^{new}$, and an upper quartile is $Q_3^{new}$, a lower quartile of an energy ratio of the previous-generation detection device in the stationary scenario is $Q_1^{old}$, a median is $Q_2^{old}$, and an upper quartile is $Q_3^{old}$. A first increase ratio $Q_1^{increase}$ of the lower quartiles is calculated based on the two lower quartiles, a second increase ratio $Q_2^{increase}$ of the medians is calculated based on the two medians, and a third increase ratio $Q_3^{increase}$ of the upper quartiles is calculated based on the two upper quartiles. Calculation formulas are respectively as follows:

$$Q_1^{increase} = \frac{Q_1^{new} - Q_1^{old}}{Q_1^{old}};$$

$$Q_2^{increase} = \frac{Q_2^{new} - Q_2^{old}}{Q_2^{old}};$$

$$Q_3^{increase} = \frac{Q_3^{new} - Q_3^{old}}{Q_3^{old}}.$$

It is determined whether the calculated first increase ratio $Q_1^{increase}$ of the lower quartiles, the calculated second increase ratio $Q_2^{increase}$ of the medians, and the calculated third increase ratio $Q_3^{increase}$ of the upper quartiles are greater than 0. If it is determined that the first increase ratio $Q_1^{increase}$ of the lower quartiles, the second increase ratio $Q_2^{increase}$ of the medians, and the third increase ratio $Q_3^{increase}$ of the upper quartiles are all greater than 0, it is determined that quality of the PPG signal collected by the new-generation detection device is improved relative to that of the previous-generation detection device. If it is determined that any one of the first increase ratio $Q_1^{increase}$ of the lower quartiles, the second increase ratio $Q_2^{increase}$ of the medians, or the third increase ratio $Q_3^{increase}$ of the upper quartiles is less than 0, it is determined that quality of the PPG signal collected by the new-generation detection device is degraded relative to that of the previous-generation detection device. If it is determined that the first increase ratio $Q_1^{increase}$ of the lower quartiles, the second increase ratio $Q_2^{increase}$ of the medians, and the third increase ratio $Q_3^{increase}$ of the upper quartiles are all equal to 0, it is determined that quality of the PPG signal collected by the new-generation detection device remains unchanged relative to that of the previous-generation detection device.

Different products are used as examples. A lower quartile of an energy ratio of a smartwatch in the stationary scenario is $Q_1^{watch}$, a median is $Q_2^{watch}$, and an upper quartile is $Q_3^{watch}$; and a lower quartile of an energy ratio of an electronic wristband in the stationary scenario is $Q1^{band}$, a median is $Q_2^{band}$, and an upper quartile is $Q_3^{band}$. A first increase ratio $Q_1^{increase}$ of the lower quartile of the smartwatch relative to that of the electronic wristband, a second increase ratio $Q_2^{increase}$ of the medians, and a third increase ratio $Q_3^{increase}$ of the upper quartiles are separately calculated. Calculation formulas are respectively as follows:

$$Q_1^{incease} = \frac{Q_1^{watch} - Q_1^{band}}{Q_1^{old}};$$

$$Q_2^{incease} = \frac{Q_2^{watch} - Q_2^{band}}{Q_2^{old}};$$

$$Q_3^{incease} = \frac{Q_3^{watch} - Q_3^{band}}{Q_3^{old}}.$$

It is determined whether the calculated first increase ratio $Q_1^{increase}$ of the lower quartiles, the calculated second increase ratio $Q_2^{increase}$ of the medians, and the calculated third increase ratio $Q_3^{increase}$ of the upper quartiles are all greater than 0. If it is determined that the first increase ratio $Q_1^{increase}$ of the lower quartiles, the second increase ratio $Q_2^{increase}$ of the medians, and the third increase ratio $Q_3^{increase}$ of the upper quartiles are all greater than 0, it is determined that quality of a PPG signal collected by the smartwatch is improved relative to that of the electronic wristband. If it is determined that any one of the first increase ratio $Q_1^{increase}$ of the lower quartiles, the second increase ratio $Q_2^{increase}$ of the medians, or the third increase ratio $Q_3^{increase}$ of the upper quartiles is less than 0, it is determined that the quality of the PPG signal collected by the smartwatch is degraded relative to that of the electronic wristband. If it is determined that the first increase ratio $Q_1^{increase}$ of the lower quartiles, the second increase ratio $Q_2^{increase}$ of the medians, and the third increase ratio $Q_3^{increase}$ of the upper quartiles are all equal to 0, it is determined that the quality of the PPG signal collected by the smartwatch remains unchanged relative to that of the electronic wristband.

In another embodiment of this application, an average value of quality evaluation parameter increase ratios may be calculated, that is, an average value of the first increase ratio $Q_1^{increase}$ of the lower quartiles, the second increase ratio $Q_2^{increase}$ of the medians, and the third increase ratio $Q_3^{increase}$ of the upper quartiles is calculated. If the average value of the quality evaluation parameter increase ratios is greater than 0, it indicates that quality of the PPG signal in the first evaluation dimension is improved relative to that in the second evaluation dimension.

In another embodiment of this application, weight values of quality evaluation parameter increase ratios may be set, and a sum of the quality evaluation parameter increase ratios may be calculated based on the weight values. If the sum of the quality evaluation parameter increase ratios is greater than 0, it indicates that quality of the PPG signal in the first evaluation dimension is improved relative to that in the second evaluation dimension. If a weight of the first increase ratio $Q_1^{increase}$ of the lower quartiles is e, a weight of the second increase ratio $Q_2^{increase}$ of the medians is f, and a weight of the third increase ratio $Q_3^{increase}$ of the upper quartiles is g, the sum of the quality evaluation parameter increase ratios is $d = e*Q_1^{increase} + f*Q_2^{increase} + g*Q_3^{increase}$.

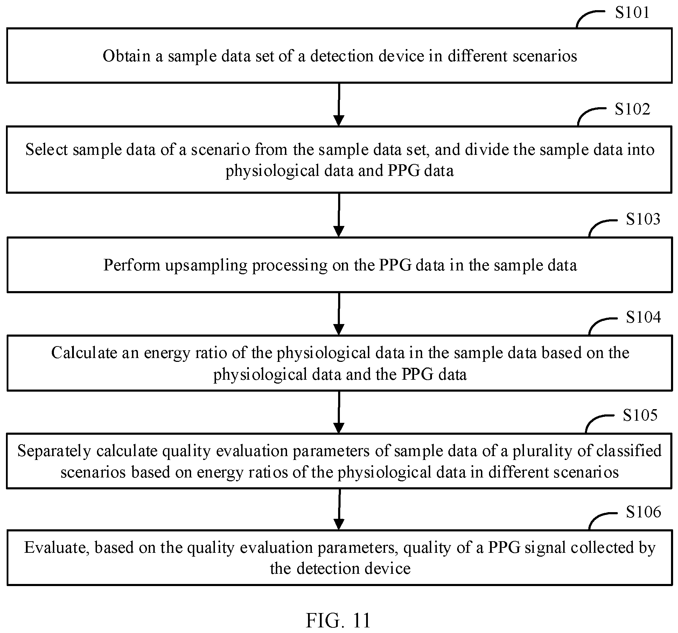

FIG. 11 is a flowchart of a physiological detection signal quality evaluation method according to an embodiment of this application. The method is applied to an electronic device 100, and specifically includes the following steps.

S101 and S102 are respectively the same as S301 and S302, and descriptions are not repeated herein.

S103: Perform upsampling processing on the PPG data $P_0$ in the sample data.

In an embodiment of this application, the performing upsampling processing on PPG data $P_0$ in the sample data includes: performing upsampling processing of a second preset multiple on the PPG data $P_0$ in the sample data. Optionally, the second preset multiple is 5 times.

Specifically, it is assumed that the PPG data $P_0$ in the sample data includes n+1 data nodes, and $P_0 = [(t_0, x_0), (t_1, x_1), ..., (t_n, x_n)]$. Quintuple quadratic interpolation upsampling is performed on the PPG data $P_0$, and PPG data obtained after upsampling is output, to obtain PPG data $P_1$ obtained after upsampling. In other words, interpolation processing may be performed on the PPG data $P_0$ by using a binary linear equation as an interpolation function.

S104: Calculate an energy ratio of the physiological data in the sample data based on the physiological data H and the PPG data $P_1$.

S105 and S106 are respectively the same as S304 and S305, and descriptions are not repeated herein.

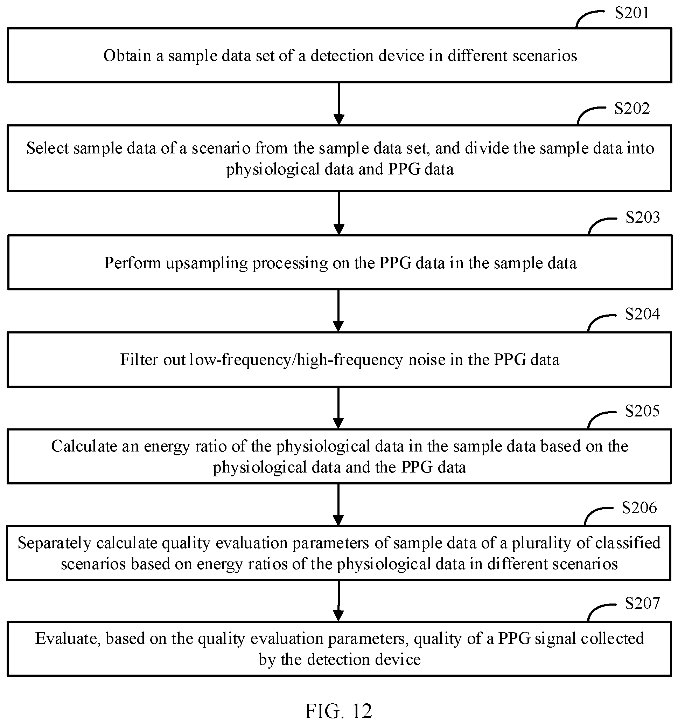

FIG. 12 is a flowchart of a physiological detection signal quality evaluation method according to another embodiment of this application. The method is applied to an electronic device 100, and specifically includes the following steps.

S201-S203 are respectively the same as S101-S103, and descriptions are not repeated herein.

S204: Filter out low-frequency/high-frequency noise in PPG data $P_1$.

In an embodiment of this application, the filtering out low-frequency/high-frequency noise in PPG data $P_1$ includes: inputting the PPG data $P_1$ into an f-order band-pass filter, and outputting filtered PPG data $P_2$ by using the f-order band-pass filter. An upper threshold of the f-order band-pass filter is $F_h$ and a lower threshold is $F_1$. The high-frequency noise in the PPG data $P_1$ is filtered out by using the upper threshold $F_h$ of the f-order band-pass filter, and the low-frequency noise in the PPG data $P_1$ is filtered out by using the lower threshold $F_1$ of the f-order band-pass filter. The order f, the upper threshold $F_h$, and the lower threshold $F_1$ may be set based on a requirement. For example, the f-order band-pass filter may be a fourth-order band-pass filter, the upper threshold $F_h$ may be 400 Hz, and the lower threshold $F_1$ is 100 Hz.

S205: Calculate an energy ratio of the physiological data in the sample data based on the physiological data H and PPG data $P_2$.

S206 and S207 are respectively the same as S105 and S106, and descriptions are not repeated herein.

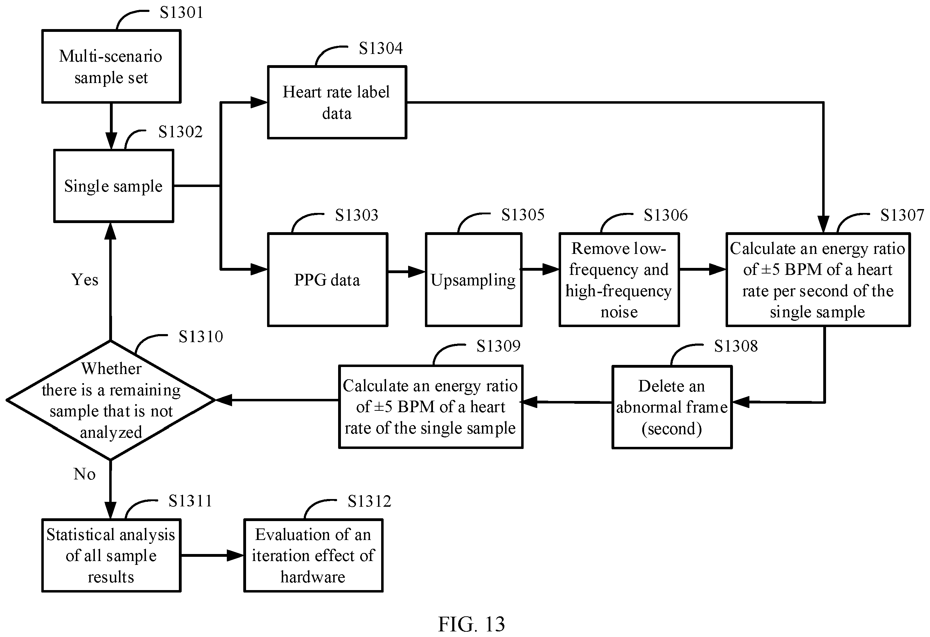

FIG. 13 is a flowchart of a physiological detection signal quality evaluation method according to another embodiment of this application. The method is applied to an electronic device 100, and specifically includes the following steps.

S1301: Collect a multi-scenario sample set.

S1302: Select a single sample.

S1303: Obtain physiological data of the single sample.

S1304: Obtain PPG data of the single sample.

S1305: Perform upsampling on the PPG data.

S1306: Filter out low-frequency and high-frequency noise in the PPG data.

S1307: Calculate an energy ratio of $\pm 5$ BPM of a heart rate per second of the single sample.

S1308: Delete an abnormal frame.

S1309: Calculate an energy ratio of $\pm 5$ BPM of a heart rate of the single sample.

S1310: Determine whether there is a remaining sample that is not analyzed. If yes, S1302 is performed. If no, S1311 is performed.

S1311: Perform statistical analysis on all sample results.

S1312: Evaluate a hardware iteration effect, and evaluate quality of PPG signals collected by hardware of different generations.

According to the physiological detection signal quality evaluation method provided in this application, an iterative baseline may be determined based on automatic statistical analysis of a multi-scenario sample group, and indicators at a hardware end and an algorithm end are synchronized at a hardware evaluation layer by using a consistent target (for example, a gold standard heart rate), to promote standardized iteration of signal quality of hardware. In the physiological detection signal quality evaluation method provided in this application, frequency-domain interpolation processing is used to increase frequency-domain resolution to further increase calculation precision of the energy ratio. In the physiological detection signal quality evaluation method provided in this application, an abnormal evaluation point in a single sample can be detected, a gold standard deviation can be corrected, and validity of the sample can be improved.

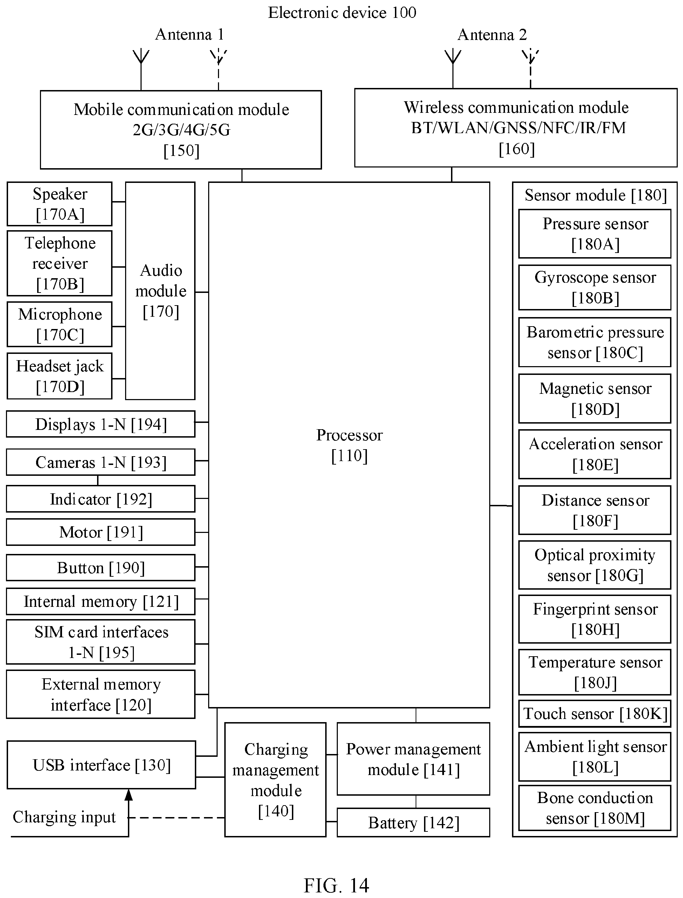

As shown in FIG. 14, the electronic device 100 may be a mobile phone, a tablet computer, a desktop computer, a laptop computer, a handheld computer, a notebook computer, an ultra-mobile personal computer (Ultra-mobile Personal Computer, UMPC), a netbook, a cellular phone, a personal digital assistant (Personal Digital Assistant, PDA), an augmented reality (Augmented Reality, AR) device, a virtual reality (Virtual Reality, VR) device, an artificial intelligence (Artificial Intelligence, AI) device, a wearable device, a vehicle-mounted device, a smart home device, and/or a smart city device. A specific type of the electronic device 100 is not specifically limited in this embodiment of this application.

The electronic device 100 may include a processor 110, an external memory interface 120, an internal memory 121, a universal serial bus (Universal Serial Bus, USB) interface 130, a charging management module 140, a power management module 141, a battery 142, an antenna 1, an antenna 2, a mobile communication module 150, a wireless communication module 160, an audio module 170, a speaker 170A, a telephone receiver 170B, a microphone 170C, a headset jack 170D, a sensor module 180, a button 190, a motor 191, an indicator 192, a camera 193, a display 194, a subscriber identity module (Subscriber Identification Module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of the present invention does not constitute a specific limitation on the electronic device 100. In some other embodiments of this application, the electronic device 100 may include more or fewer components than those shown in the figure, combine some components, split some components, or have different component arrangements. The components shown in the figure may be implemented by hardware, software, or a combination of software and hardware.

The processor 110 may include one or more processing units. For example, the processor 110 may include an application processor (Application Processor, AP), a modem processor, a graphics processing unit (Graphics Processing Unit, GPU), an image signal processor (Image Signal Processor, ISP), a controller, a video codec, a digital signal processor (Digital Signal Processor, DSP), a baseband processor, and/or a neural-network processing unit (Neural-network Processing Unit, NPU). Different processing units may be independent components, or may be integrated into one or more processors.

The controller may generate an operation control signal based on instruction operation code and a timing signal, to complete control of instruction fetching and instruction execution.

A memory may be further disposed in the processor 110, to store instructions and data. In some embodiments, the memory in the processor 110 is a cache. The memory may store instructions or data that is recently used or cyclically used by the processor 110. If the processor 110 needs to use the instructions or the data again, the processor 110 may directly invoke the instructions or the data from the memory. This avoids repeated access and reduces a waiting time of the processor 110, thereby improving system efficiency.

In some embodiments, the processor 110 may include one or more interfaces. The interface may include an inter-integrated circuit (Inter-Integrated Circuit, I2C) interface, an inter-integrated circuit sound (Inter-integrated Circuit Sound, I2S) interface, a pulse code modulation (Pulse Code Modulation, PCM) interface, a universal asynchronous receiver/-transmitter (universal asynchronous receiver/transmitter, UART) interface, a mobile industry processor interface (Mobile Industry Processor Interface, MIPI), a general-purpose input/output (General-Purpose Input/Output, GPIO) interface, a subscriber identity module (Subscriber Identity Module, SIM) interface, a universal serial bus (Universal Serial Bus, USB) interface, and/or the like.

The I2C interface is a bidirectional synchronous serial bus, including a serial data line (Serial Data Line, SDA) and a serial clock line (Derail Clock Line, SCL). In some embodiments, the processor 110 may include a plurality of I2C buses. The processor 110 may be separately coupled to the touch sensor 180K, a charger, a flashlight, the camera 193, and the like through different I2C bus interfaces. For example, the processor 110 may be coupled to the touch sensor 180K through the I2C interface, so that the processor 110 communicates with the touch sensor 180K through the I2C bus interface, thereby implementing a touch function of the electronic device 100.

The I2S interface may be used for audio communication. In some embodiments, the processor 110 may include a plurality of I2S buses. The processor 110 may be coupled to the audio module 170 through the I2S bus, to implement communication between the processor 110 and the audio module 170. In some embodiments, the audio module 170 may transfer an audio signal to the wireless communication module 160 through the I2S interface, to implement a function of answering a call through a Bluetooth headset.

The PCM interface may also be used for audio communication, to sample, quantize, and encode an analog signal. In some embodiments, the audio module 170 may be coupled to the wireless communication module 160 through a PCM bus interface. In some embodiments, the audio module 170 may also transfer an audio signal to the wireless communication module 160 through the PCM interface, to perform the function of answering a call through the Bluetooth headset. Both the I2S interface and the PCM interface may be used for audio communication.

The UART interface is a universal serial data bus used for asynchronous communication. The bus may be a bidirectional communication bus. The bus converts to-be-transmitted data between serial communication and parallel communication. In some embodiments, the UART interface is usually configured to connect the processor 110 and the wireless communication module 160. For example, the processor 110 communicates with a Bluetooth module in the wireless communication module 160 through the UART interface, to implement a Bluetooth function. In some embodiments, the audio module 170 may transfer an audio signal to the wireless communication module 160 through the UART interface, to implement a function of playing music through the Bluetooth headset.

The MIPI interface may be configured to connect the processor 110 and peripheral devices such as the display 194 and the camera 193. The MIPI interface include a camera serial interface (Camera Serial Interface, CSI), a display serial interface (Display Serial Interface, DSI), and the like. In some embodiments, the processor 110 and the camera 193 communicate with each other through the CSI interface, to implement a photographing function of the electronic device 100. The processor 110 and the display 194 communicate with each other through the DSI interface, to implement a display function of the electronic device 100.

The GPIO interface may be configured by using software. The GPIO interface may be configured as a control signal or may be configured as a data signal. In some embodiments, the GPIO interface may be configured to connect the processor 110 and the camera 193, the display 194, the wireless communication module 160, the audio module 170, the sensor module 180, or the like. The GPIO interface may alternatively be configured as the I2C interface, the I2S interface, the UART interface, the MIPI interface, or the like.

The USB interface 130 is an interface that complies with USB standard specifications, and may be specifically a Mini USB interface, a Micro USB interface, a USB Type C interface, or the like. The USB interface 130 may be configured to be connected to the charger to charge the electronic device 100, or may be configured to transmit data between the electronic device 100 and a peripheral device, or may be configured to be connected to a headset, to play audio by using the headset. The interface may be further configured to connect to another electronic device 100, for example, an AR device.

It may be understood that an interface connection relationship between modules illustrated in this embodiment of the present invention is merely an example for description, and does not constitute a limitation on the structure of the electronic device 100. In some other embodiments of this application, different interface connection manners in the foregoing embodiments or a combination of a plurality of interface connection manners may alternatively be used for the electronic device 100.

The charging management module 140 is configured to receive a charging input from the charger. The charger may be a wireless charger or a wired charger. In some embodiments of wired charging, the charging management module 140 may receive a charging input of a wired charger by using the USB interface 130. In some embodiments of wireless charging, the charging management module 140 may receive a wireless charging input through a wireless charging coil of the electronic device 100. When charging the battery 142, the charging management module 140 may further supply power to the electronic device 100 by using the power management module 141.

The power management module 141 is configured to be connected to the battery 142, the charging management module 140, and the processor 110. The power management module 141 receives an input of the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a quantity of battery cycles, and a battery health status (leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device 100 may be implemented by using the antenna 1, the antenna 2, the mobile communication module 150, the wireless communication module 160, the modem processor, the baseband processor, and the like.

The antenna 1 and the antenna 2 are configured to transmit and receive electromagnetic wave signals. Each antenna in the electronic device 100 may be configured to cover one or more communication frequency bands. Different antennas may be further multiplexed to improve antenna utilization. For example, the antenna 1 may be multiplexed as a diversity antenna of a wireless local area network. In some other embodiments, the antenna may be used in combination with a tuning switch.

The mobile communication module 150 may provide a solution for wireless communication including 2G/3G/4G/5G and the like applied to the electronic device 100. The mobile communication module 150 may include at least one filter, a switch, a power amplifier, a low noise amplifier (Low Noise Amplifier, LNA), and the like. The mobile communication module 150 may receive an electromagnetic wave by using the antenna 1, perform processing such as filtering and amplification on the received electromagnetic wave, and send a processed electromagnetic wave to the modem processor for demodulation. The mobile communication module 150 may further amplify a signal obtained after modulation by the modem processor, and convert, by using the antenna 1, an amplified signal into an electromagnetic wave for radiation. In some embodiments, at least some functional modules in the mobile communication module 150 may be disposed in the processor 110. In some embodiments, at least some functional modules in the mobile communication module 150 may be disposed in a same device as at least some modules in the processor 110.

The modem processor may include a modulator and a demodulator. The modulator is configured to modulate a to-be-sent low-frequency baseband signal into an intermediate- or high-frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transferred to the application processor. The application processor outputs a sound signal by using an audio device (not limited to the speaker 170A or the telephone receiver 170B), or displays an image or a video by using the display 194. In some embodiments, the modem processor may be a separate device. In some other embodiments, the modem processor may be independent of the processor 110, and the modem processor and the mobile communication module 150 or another functional module are disposed in a same device.

The wireless communication module 160 may provide a solution for wireless communication including a wireless local area network (Wireless Local Area Networks, WLAN) (such as a wireless fidelity (Wireless Fidelity, Wi-Fi) network), Bluetooth (Bluetooth, BT), and a global navigation satellite system (Global Navigation Satellite System, GNSS), frequency modulation (Frequency Modulation, FM), a near field communication (Near Field Communication, NFC) technology, an infrared (Infrared, IR) technology, and the like applied to the electronic device 100. The wireless communication module 160 may be one or more devices integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 100 are coupled, and the antenna 2 and the wireless communication module 160 are coupled, so that the electronic device 100 can communicate with a network and another device by using a wireless communication technology. The wireless communications technology may include a global system for mobile communications (Global System For Mobile Communications, GSM), a general packet radio service (General Packet Radio Service, GPRS), code division multiple access (Code Division Multiple Access, CDMA), wideband code division multiple access (Wideband Code Division Multiple Access, WCDMA), time-division code division multiple access (Time-Division Code Division Multiple Access, TD-SCDMA), long term evolution (Long Term Evolution, LTE), BT, a GNSS, a WLAN, NFC, FM, an IR technology, and/or the like. The GNSS may include a global positioning system (Global Positioning System, GPS), a global navigation satellite system (Global Navigation Satellite System, GLONASS), a Beidou navigation satellite system (Beidou Navigation Satellite System, BDS), a quasi-zenith satellite system (Quasi-Zenith Satellite System, QZSS), and/or a satellite based augmentation system (Satellite Based Augmentation Systems, SBAS).

The electronic device 100 implements a display function by using the GPU, the display 194, the application processor, and the like. The GPU is a microprocessor for image processing, and is connected to the display 194 and the application processor. The GPU is configured to perform mathematical and geometric computing for graphics rendering. The processor 110 may include one or more GPUs, and the GPU executes a program instruction to generate or change display information.

The display 194 is configured to display an image, a video, or the like. The display 194 includes a display panel. The display panel may be a liquid crystal display (Liquid Crystal Display, LCD), an organic light-emitting diode (Organic Light-Emitting Diode, OLED), an active-matrix organic light emitting diode (Active-Matrix Organic Light Emitting Diode, AMOLED), a flexible light-emitting diode (Flex Light-Emitting Diode, FLED), a Miniled, a Microled, a Micro-OLED, a quantum dot light emitting diode (Quantum Dot Light Emitting Diodes, QLED), or the like. In some embodiments, the electronic device 100 may include one or N displays 194, where N is a positive integer greater than 1.

The electronic device 100 may implement a photographing function by using the ISP, the camera 193, the video codec, the GPU, the display 194, the application processor, and the like.

The ISP is configured to process data fed back by the camera 193. For example, during photographing, a shutter is pressed, light is transmitted to a photosensitive element of the camera through a lens, an optical signal is converted into an electrical signal, and the photosensitive element of the camera transmits the electrical signal to the ISP for processing, to convert the electrical signal into an image visible to a naked eye. The ISP may further perform algorithm optimization on noise, brightness, and complexion of the image. The ISP may further optimize parameters such as exposure and color temperature of a shooting scene. In some embodiments, the ISP may be disposed in the camera 193.

The camera 193 is configured to capture a still image or a video. An optical image is generated for an object by using the lens and is projected onto the photosensitive element. The photosensitive element may be a charge coupled device (Charge Coupled Device, CCD) or a complementary metal-oxide-semiconductor (Complementary Metal-Oxide-Semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format, for example, RGB or YUV. In some embodiments, the electronic device 100 may include one or N cameras 193, where N is a positive integer greater than 1.

The digital signal processor is configured to process a digital signal. In addition to processing a digital image signal, the digital signal processor can further process another digital signal. For example, when the electronic device 100 selects a frequency, the digital signal processor is configured to perform Fourier transform and the like on frequency energy.

The video codec is configured to compress or decompress a digital video. The electronic device 100 may support one or more video codecs. Therefore, the electronic device 100 may play or record videos in a plurality of encoding formats, such as moving picture experts group (moving picture experts group, MPEG)1, MPEG2, MPEG3, and MPEG4.

The NPU is a neural-network (Neural-Network, NN) computing processor. By referring to a structure of a biological neural network, for example, to a transmission mode between neurons in a human-brain, the NPU quickly processes input information and is also capable of continuous self-learning. Applications such as intelligent cognition of the electronic device 100, for example, image recognition, face recognition, speech recognition, and text understanding, may be implemented by using the NPU.

The internal memory 121 may include one or more random access memories (Random Access Memory, RAM) and one or more non-volatile memories (Non-Volatile Memory, NVM).

The random access memory may include a static random access memory (Static Random-Access Memory, SRAM), a dynamic random access memory (Dynamic Random Access Memory, DRAM), a synchronous dynamic random access memory (Synchronous Dynamic Random Access Memory, SDRAM), a double data rate synchronous dynamic random access memory (Double Data Rate Synchronous Dynamic Random Access Memory, DDR SDRAM, for example, a fifth-generation DDR SDRAM, which is generally referred to as a DDR5 SDRAM), and the like.

The non-volatile memory may include a magnetic disk storage device and a flash memory (flash memory).

The flash memory may be classified, based on an operating principle, into an NOR FLASH, an NAND FLASH, a 3D NAND FLASH, and the like; may be classified, based on a quantity of electric potential levels of a cell, into a single-level cell (Single-Level Cell, SLC), a multi-level cell (Multi-Level Cell, MLC), a triple-level cell (Triple-Level Cell, TLC), a quad-level cell (Quad-Level Cell, QLC), and the like; or may be classified, based on a storage specification, into a universal flash storage (Universal Flash Storage, UFS), an embedded multimedia card (Embedded Multi Media Card, eMMC), and the like.

The random access memory may be directly read and written by the processor 110, and may be configured to store an executable program (for example, a machine instruction) of an operating system or another running program, and may be further configured to store data of a user and data of an application, and the like.

The non-volatile memory may further store the executable program, the data of the user and the application, and the like, which may be loaded into the random access memory in advance for directly reading and writing by the processor 110.

The external memory interface 120 may be configured to connect to an external non-volatile memory, to expand a storage capability of the electronic device 100. The external non-volatile memory communicates with the processor 110 by using the external memory interface 120, to implement a data storage function. For example, files such as music and a video are stored in the external non-volatile memory.

The internal memory 121 or the external memory interface 120 is configured to store one or more computer programs. One or more computer programs are configured to be executed by the processor 110. The one or more computer programs include a plurality of instructions. When the plurality of instructions are executed by the processor 110, the physiological detection signal quality evaluation method performed on the electronic device 100 in the foregoing embodiments may be implemented, to implement a physiological detection signal quality evaluation display function of the electronic device 100.

The electronic device 100 may implement audio functions such as music playing and sound recording by using the audio module 170, the speaker 170A, the telephone receiver 170B, the microphone 170C, the headset jack 170D, the application processor, and the like.

The audio module 170 is configured to convert digital audio information into an analog audio signal for output, and is also configured to convert analog audio input into a digital audio signal. The audio module 170 may be further configured to encode and decode an audio signal. In some embodiments, the audio module 170 may be disposed in the processor 110, or some functional modules in the audio module 170 may be disposed in the processor 110.

The speaker 170A, also referred to as a "loudspeaker", is configured to convert an audio electrical signal into a sound signal. The electronic device 100 may be configured to listen to music or answer a call in a hands-free mode by using the speaker 170A.

The telephone receiver 170B, also referred to as an "earpiece", is configured to convert an audio electrical signal into a sound signal. When a call is answered or audio information is listened to by using the electronic device 100, the telephone receiver 170B may be put close to a human ear to listen to a voice.

The microphone 170C, also referred to as a "mic" or "mike", is configured to convert a sound signal into an electrical signal. When making a call or sending voice information, the user may make a sound by approaching the mouth to the microphone 170C, to input a sound signal to the microphone 170C. At least one microphone 170C may be disposed in the electronic device 100. In some other embodiments, two microphones 170C may be disposed in the electronic device 100, to implement a noise reduction function in addition to collecting a sound signal. In some other embodiments, three, four, or more microphones 170C may be disposed in the electronic device 100, to collect a sound signal, implement noise reduction, and identify a sound source, so as to implement a directional recording function and the like.

The headset jack 170D is configured to be connected to a wired headset. The headset jack 170D may be the USB interface 130, or may be a 3.5 mm Open Mobile Terminal Platform 100 (Open Mobile Terminal Platform, OMTP) standard interface, or a cellular telecommunications industry association of the USA (Cellular Telecommunications Industry Association of the USA, CTIA) standard interface.

The pressure sensor 180A is configured to sense a pressure signal, and can convert the pressure signal into an electrical signal. In some embodiments, the pressure sensor 180A may be disposed on the display 194. There are many types of pressure sensors 180A, for example, a resistive pressure sensor, an inductive pressure sensor, and a capacitive pressure sensor. The capacitive pressure sensor may include at least two parallel plates having an electrically conductive material. When force is applied onto the pressure sensor 180A, a capacitance between electrodes changes. The electronic device 100 determines strength of pressure based on a change of the capacitance. When a touch operation is performed on the display 194, the electronic device 100 detects intensity of the touch operation by using the pressure sensor 180A. The electronic device 100 may also calculate a touched location based on a detected signal of the pressure sensor 180A. In some embodiments, touch operations acting on a same touch location but having different touch operation intensity may correspond to different operation instructions. For example, when a touch operation whose touch operation intensity is less than a first pressure threshold is performed on an SMS message application icon, an instruction for viewing an SMS message is executed. For example, when a touch operation whose touch operation intensity is greater than or equal to the first pressure threshold is performed on the SMS message application icon, an instruction for creating a new SMS message is executed.

The gyroscope sensor 180B may be configured to determine a moving posture of the electronic device 100. In some embodiments, angular velocities of the electronic device 100 around three axes (namely, an x axis, a y axis, and a z axis) may be determined by using the gyroscope sensor 180B. The gyroscope sensor 180B may be configured to implement image stabilization during photographing. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may be further used in a navigation scenario and a motion sensing game scenario.

The barometric pressure sensor 180C is configured to measure atmospheric pressure. In some embodiments, the electronic device 100 calculates an altitude by using a barometric pressure value measured by the barometric pressure sensor 180C, to assist in positioning and navigation.

The magnetic sensor 180D may include a Hall sensor. The electronic device 100 may detect opening and closing of a flip leather case by using the magnetic sensor 180D. In some embodiments, when the electronic device 100 is a flip phone, the electronic device 100 may detect opening and closing of a flip cover based on the magnetic sensor 180D, and further set features such as automatic unlocking of the flip cover based on a detected opening and closing state of the leather case or a detected opening and closing state of the flip cover.

The acceleration sensor 180E may detect magnitudes of acceleration of the electronic device 100 in all directions (generally in three axes), and may detect a magnitude and a direction of gravity when the electronic device 100 is still. The acceleration sensor 180E may be further configured to recognize a posture of the electronic device 100, and is applied to switching between a landscape mode and a portrait mode, a pedometer, or another application.

The distance sensor 180F is configured to measure a distance. The electronic device 100 may measure a distance by using infrared or laser. In some embodiments, in a photographing scenario, the electronic device 100 may measure a distance by using the distance sensor 180F, to implement quick focusing.

The optical proximity sensor 180G may include, for example, a light-emitting diode (LED) and an optical detector such as a photodiode. The light-emitting diode may be an infrared light-emitting diode. The electronic device 100 emits infrared light to the outside by using the light-emitting diode. The electronic device 100 uses the photodiode to detect reflected infrared light from a nearby object. When sufficient reflected light is detected, it may be determined that there is an object near the electronic device 100. When detecting insufficient reflected light, the electronic device 100 may determine that there is no object near the electronic device 100. The electronic device 100 may detect, by using the optical proximity sensor 180G, that the user holds the electronic device 100 close to an ear for a call, to implement automatic screen-off to save power. The optical proximity sensor 180G may alternatively be used in a leather case mode or a pocket mode to automatically unlock or lock a screen.

The ambient light sensor 180L is configured to sense luminance of ambient light. The electronic device 100 may adaptively adjust luminance of the display 194 based on the sensed luminance of the ambient light. The ambient light sensor 180L can also be configured to automatically adjust white balance during shooting. The ambient light sensor 180L can further cooperate with the optical proximity sensor 180G to detect whether the electronic device 100 is in a pocket, to prevent accidental touch.

The fingerprint sensor 180H is configured to collect a fingerprint. The electronic device 100 may implement fingerprint unlock, application lock accessing, fingerprint-based photographing, fingerprint-based call answering, and the like by using a feature of the collected fingerprint.

The temperature sensor 180J is configured to detect temperature. In some embodiments, the electronic device 100 executes a temperature processing policy by using the temperature detected by the temperature sensor 180J. For example, when the temperature reported by the temperature sensor 180J exceeds a threshold, the electronic device 100 degrades performance of a processor located near the temperature sensor 180J, to reduce power consumption and implement thermal protection. In some other embodiments, when the temperature is lower than another threshold, the electronic device 100 heats the battery 142, to prevent the electronic device 100 from being abnormally powered off due to low temperature. In some other embodiments, when the temperature is lower than still another threshold, the electronic device 100 boosts an output voltage of the battery 142, to avoid abnormal power-off caused by low temperature.

The touch sensor 180K is also referred to as a "touch component". The touch sensor 180K may be disposed in the display 194. The touch sensor 180K and the display 194 constitute a touchscreen, which is also referred to as a "touch control screen". The touch sensor 180K is configured to detect a touch operation performed on or near the touch sensor 180K. The touch sensor may transmit the detected touch operation to the application processor, to determine a type of a touch event. A visual output related to the touch operation may be provided by using the display 194. In some other embodiments, the touch sensor 180K may be alternatively disposed on a surface of the electronic device 100, and is at a location different from that of the display 194.

The bone conduction sensor 180M may obtain a vibration signal. In some embodiments, the bone conduction sensor 180M may obtain a vibration signal of a vibration bone in human vocal-cord part. The bone conduction sensor 180M may also be in contact with a human pulse to receive a blood pressure beating signal. In some embodiments, the bone conduction sensor 180M may alternatively be disposed in a headset, to form a bone conduction headset in combination with the headset. The audio module 170 may obtain a speech signal through parsing based on the vibration signal that is of the vibration bone in the vocal-cord part and that is obtained by the bone conduction sensor 180M, to implement a speech function. The application processor may parse heart rate information based on the blood pressure beating signal obtained by the bone conduction sensor 180M, to implement a heart rate detection function.

The button 190 includes a power on/off button, a volume button, and the like. The button 190 may be a mechanical button, or may be a touch button. The electronic device 100 may receive a key input and generate a key signal input related to user settings and function control of the electronic device 100.

The motor 191 may generate a vibration prompt. The motor 191 may be configured to provide a vibration prompt for an incoming call, and may also be configured to provide vibration feedback for a touch. For example, touch operations performed on different applications (such as photographing and audio playing) may correspond to different vibration feedback effects. For touch operations performed in different regions of the display 194, the motor 191 may also correspond to different vibration feedback effects. Different application scenarios (for example, a time prompt, information receiving, an alarm clock, and a game) may also correspond to different vibration feedback effects. A touch vibration feedback effect may be further customized.

The indicator 192 may be an indicator light, may be configured to indicate a charging status or a power change, and may be further configured to indicate a message, a missed incoming call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support 1 or N SIM card interfaces, where N is a positive integer greater than 1. The SIM card interface 195 may support a Nano SIM card, a Micro SIM card, a SIM card, or the like. A plurality of cards may be simultaneously inserted into a same SIM card interface 195. The plurality of cards may be of a same type or different types. The SIM card interface 195 may also be compatible with SIM cards of different types. The SIM card interface 195 may be further compatible with the external storage card. The electronic device 100 interacts with a network by using the SIM card, to implement functions such as a call and data communication. In some embodiments, the electronic device 100 uses an eSIM, that is, an embedded SIM card. The eSIM card may be embedded in the electronic device 100 and cannot be separated from the electronic device 100.

An embodiment further provides a computer storage medium. The computer storage medium stores computer instructions. When the computer instructions are run on an electronic device 100, the electronic device 100 is enabled to perform the foregoing related method steps to implement the physiological detection signal quality evaluation method in the foregoing embodiments.

An embodiment further provides a computer program product. When the computer program product runs on a computer, the computer is enabled to perform the foregoing related steps to implement the physiological detection signal quality evaluation method in the foregoing embodiments.

In addition, an embodiment of this application further provides an apparatus. The apparatus may be specifically a chip, a component, or a module. The apparatus may include a processor and a memory that are connected to each other. The memory is configured to store computer-executable instructions. When the apparatus runs, the processor may execute the computer-executable instructions stored in the memory, so that the chip performs the physiological detection signal quality evaluation method in the foregoing method embodiments.

The electronic device, the computer storage medium, the computer program product, or the chip provided in the embodiments is configured to perform the corresponding method provided above. Therefore, for beneficial effects that can be achieved by the electronic device, the computer storage medium, the computer program product, or the chip, refer to beneficial effects in the corresponding method provided above. Details are not described herein again.

Through the descriptions of the foregoing implementations, a person skilled in the art may clearly understand that, for the purpose of convenient and brief description, only division of the foregoing functional modules is used as an example for description. In actual application, the functions may be allocated to and completed by different functional modules as required. In other words, an internal structure of the apparatus is divided into different functional modules, to complete all or some of the functions described above.

In several embodiments provided in this application, it should be understood that the disclosed apparatus and method may be implemented in another manner. For example, the described apparatus embodiments are merely examples. For example, division into the modules or units is merely logical function division. In actual implementation, there may be another division manner. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may be one or more physical units, that is, may be located in one place, or may be distributed in a plurality of different places. Some or all of the units may be selected according to actual requirements to achieve the purpose of the solutions in the embodiments.

In addition, functional units in embodiments of this application may be integrated into one processing unit, or each of the units may exist alone physically, or two or more units are integrated into one unit. The integrated unit may be implemented in a form of hardware, or may be implemented in a form of a software functional unit.

When the integrated unit is implemented in a form of a software functional unit and sold or used as an independent product, the integrated unit may be stored in a readable storage medium. Based on such an understanding, the technical solutions in embodiments of this application essentially, or the part contributing to the conventional technology, or all or some of the technical solutions may be implemented in a form of a software product. The software product is stored in a storage medium and includes several instructions for instructing a device (which may be a single-chip microcomputer, a chip, or the like) or a processor (processor) to perform all or some of the steps of the methods in embodiments of this application. The foregoing storage medium includes any medium that can store program code, such as a USB flash drive, a removable hard disk, a read-only memory (Read-Only Memory, ROM), a random access memory (Random Access Memory, RAM), a magnetic disk, or an optical disc.

**Claims**

1. A physiological detection signal quality evaluation method, wherein the method comprises:

    obtaining (S301) a sample data set collected by a detection device (200) in different scenarios;
    selecting (S302) sample data of a scenario from the sample data set, and dividing the sample data into physiological data and PPG data;
    calculating (S303) an energy ratio of the physiological data based on the physiological data and the PPG data;
    separately calculating (S304) quality evaluation parameters of sample data of a plurality of classified scenarios based on energy ratios of the physiological data corresponding to different scenarios; and
    evaluating (305), based on the quality evaluation parameters, quality of a physiological detection signal collected by the detection device (200); **characterised in that** calculating (S303) the energy ratio of the physiological data based on the physiological data and the PPG data comprises:

        separately calculating, based on the physiological data and the PPG data, a plurality of energy ratios of frequencies corresponding to a plurality of pieces of physiological data; and
        calculating an average value of the plurality of energy ratios of the frequencies corresponding to the plurality of pieces of physiological data, and using the average value as the energy ratio of the physiological data.

2. The physiological detection signal quality evaluation method according to claim 1, wherein the obtaining (S301) the sample data set collected by the detection device (200) in different scenarios comprises:

    setting (S3011) a plurality of preset scenarios based on a label;
    connecting (S3012) the detection device (200) to a data collection end;
    collecting (S3013), by the detection device (200), data based on a single preset scenario to generate a single piece of sample data; and

outputting (S3014) a multi-scenario sample data set based on sample data of the plurality of preset scenarios.

3. The physiological detection signal quality evaluation method according to claim 1, wherein the separately calculating, based on the physiological data and the PPG data, the plurality of energy ratios of frequencies corresponding to the plurality of pieces of physiological data comprises:

performing (S3031) short-time Fourier transform on the PPG data to obtain power spectral density data of the PPG data, wherein a window type of the short-time Fourier transform is a Hamming window;
determining (S3033) time points corresponding to center locations of a plurality of Hamming windows, and obtaining physiological data corresponding to each time point;
converting (S3034) the physiological data into a frequency range;
calculating (S3035), based on the power spectral density data, an energy ratio corresponding to the frequency range; and
outputting (S3036) an energy ratio list of the plurality of pieces of physiological data in the sample data based on energy ratios corresponding to a plurality of frequency ranges.

4. The physiological detection signal quality evaluation method according to claim 3, wherein the calculating (S3035), based on the power spectral density data, the energy ratio corresponding to the frequency range comprises:
dividing a sum of energy values corresponding to a plurality of frequency values in the frequency range by a sum of energy values corresponding to all frequency values to obtain the energy ratio corresponding to the frequency range.

5. The physiological detection signal quality evaluation method according to claim 3, wherein the separately calculating, based on the physiological data and the PPG data, the plurality of energy ratios of frequencies corresponding to the plurality of pieces of physiological data further comprises:
performing interpolation processing on the power spectral density data in a time-domain zero-filling or frequency-domain padding manner.

6. The physiological detection signal quality evaluation method according to claim 3, wherein the separately calculating, based on the physiological data and the PPG data, the plurality of energy ratios of frequencies corresponding to the plurality of pieces of physiological data further comprises:
deleting abnormal data in the energy ratio list of the plurality of pieces of physiological data; wherein the deleting abnormal data in the energy ratio list of the plurality of pieces of physiological data comprises:

calculating an upper quartile $Q_1^E$ and a lower quartile $Q_3^E$ in the energy ratio list;
calculating an upper threshold $U_1$ and a lower threshold $U_2$ of the abnormal data;
determining that data that is in the energy ratio list and that is less than or equal to the lower threshold or greater than or equal to the upper threshold is the abnormal data; and
outputting an energy ratio list from which the abnormal data is deleted.

7. The physiological detection signal quality evaluation method according to claim 1, wherein the quality evaluation parameters comprise a lower quartile, a median, and an upper quartile of physiological data energy ratios of a plurality of scenarios in the classified scenarios; wherein the separately calculating quality evaluation parameters of sample data of a plurality of classified scenarios based on energy ratios of the physiological data corresponding to different scenarios comprises:

classifying (S3041) the different scenarios to determine the plurality of classified scenarios;
obtaining (S3041), based on an analysis object, a plurality of physiological data energy ratios of classified scenarios separately corresponding to at least two evaluation dimensions; and
calculating (S3043) a lower quartile, a median, and an upper quartile of the physiological data energy ratios of classified scenarios separately corresponding to the at least two evaluation dimensions.

8. The physiological detection signal quality evaluation method according to claim 7, wherein the evaluating, based on the quality evaluation parameters, the quality of the physiological detection signal collected by the detection device (200) comprises:
determining, based on an increase or decrease ratio of a quality evaluation parameter of a classified scenario corresponding to a first evaluation dimension to a quality evaluation parameter of a classified scenario corresponding to a second evaluation dimension, whether the quality of the physiological detection signal collected by the detection device (200) is improved or degraded.

9. The physiological detection signal quality evaluation method according to claim 8, wherein the determining, based on the increase or decrease ratio of the quality evaluation parameter of the classified scenario corresponding to thea first evaluation dimension to the quality evaluation parameter of the classified scenario corresponding to the second evaluation dimension, whether the quality of the physiological detection signal collected by the detection device (200) is improved or degraded comprises:

calculating a lower quartile increase ratio, a median increase ratio, and an upper quartile increase ratio of a plurality of physiological data energy ratios of the classified scenario corresponding to the first evaluation dimension to a plurality of physiological data energy ratios of the classified scenario corresponding to the second evaluation dimension; and
determining, based on the lower quartile increase ratio, the median increase ratio, and the upper quartile increase ratio, whether the quality of the physiological detection signal collected by the detection device (200) is improved or degraded.

10. The physiological detection signal quality evaluation method according to claim 9, wherein the determining, based on the lower quartile increase ratio, the median increase ratio, and the upper quartile increase ratio, whether the quality of the physiological detection signal collected by the detection device (200) is improved or degraded comprises:

determining whether the lower quartile increase ratio, the median increase ratio, and the upper quartile increase ratio are greater than o; and
if it is determined that the lower quartile increase ratio, the median increase ratio, and the upper quartile increase ratio are greater than 0, determining that quality of a physiological detection signal collected by the detection device (200) in the first evaluation dimension is improved relative to that in the second evaluation dimension;
if it is determined that the lower quartile increase ratio, the median increase ratio, and the upper quartile increase ratio are all equal to 0, determining that quality of a physiological detection signal collected by the detection device (200) in the first evaluation dimension remains unchanged relative to that in the second evaluation dimension; or
if it is determined that any one of the lower quartile increase ratio, the median increase ratio, and the upper quartile increase ratio is less than 0, determining that quality of a physiological detection signal collected by the detection device (200) in the first evaluation dimension is degraded relative to that in the second evaluation dimension.

11. The physiological detection signal quality evaluation method according to claim 9, wherein the determining, based on the lower quartile increase ratio, the median increase ratio, and the upper quartile increase ratio, whether the quality of the physiological detection signal collected by the detection device (200) is improved or degraded comprises:

calculating an average value of the lower quartile increase ratio, the median increase ratio, and the upper quartile increase ratio;
determining whether the average value is greater than o; and
if it is determined that the average value is greater than 0, determining that quality of a physiological detection signal collected by the detection device (200) in the first evaluation dimension is improved relative to that in the second evaluation dimension;
if it is determined that the average value is equal to 0, determining that quality of a physiological detection signal collected by the detection device (200) in the first evaluation dimension remains unchanged relative to that in the second evaluation dimension; or
if it is determined that the average value is less than 0, determining that quality of a physiological detection signal collected by the detection device (200) in the first evaluation dimension is degraded relative to that in the second evaluation dimension.

12. The physiological detection signal quality evaluation method according to claim 9, wherein the determining, based on the lower quartile increase ratio, the median increase ratio, and the upper quartile increase ratio, whether the quality of the physiological detection signal collected by the detection device (200) is improved or degraded comprises:

separately setting weight values of the lower quartile increase ratio, the median increase ratio, and the upper quartile increase ratio;
calculating a sum of the lower quartile increase ratio, the median increase ratio, and the upper quartile increase ratio based on the weight values of the lower quartile increase ratio, the median increase ratio, and the upper quartile increase ratio;
determining whether the sum of the lower quartile increase ratio, the median increase ratio, and the upper quartile increase ratio is greater than o; and

if it is determined that the sum of the lower quartile increase ratio, the median increase ratio, and the upper quartile increase ratio is greater than 0, determining that quality of a physiological detection signal collected by the detection device (200) in the first evaluation dimension is improved relative to that in the second evaluation dimension;

if it is determined that the sum of the lower quartile increase ratio, the median increase ratio, and the upper quartile increase ratio is equal to 0, determining that quality of a physiological detection signal collected by the detection device (200) in the first evaluation dimension remains unchanged relative to that in the second evaluation dimension; or

if it is determined that the sum of the lower quartile increase ratio, the median increase ratio, and the upper quartile increase ratio is less than 0, determining that quality of a physiological detection signal collected by the detection device (200) in the first evaluation dimension is degraded relative to that in the second evaluation dimension.

13. The physiological detection signal quality evaluation method according to claim 1, wherein the method further comprises:

setting an upper threshold and a lower threshold of a band-pass frequency by using an f-order band-pass filter; and

inputting the PPG data into the band-pass filter, and filtering out the low-frequency and/or high-frequency noise in the PPG data based on the upper threshold and the lower threshold by using the band-pass filter.

14. An electronic device (100), wherein the electronic device (100) comprises a memory (121) and a processor (110);

the memory (121) is configured to store program instructions; and

the processor (110) is configured to read and execute the program instructions stored in the memory (121), and when the program instructions are executed by the processor (110), the electronic device (100) is enabled to perform the physiological detection signal quality evaluation method according to any one of claims 1 to 13.

**Patentansprüche**

1. Ein Verfahren zur Bewertung der Signalqualität physiologischer Detektion, wobei das Verfahren umfasst:

Erhalten (S301) eines von einer Detektionsvorrichtung (200) in verschiedenen Szenarien gesammelten Beispieldatensatzes;

Auswählen (S302) von Beispieldaten eines Szenarios aus dem Beispieldatensatz und Aufteilen der Beispieldaten in physiologische Daten und PPG-Daten;

Berechnen (S303) eines Energieratios der physiologischen Daten basierend auf den physiologischen Daten und den PPG-Daten;

Separates Berechnen (S304) von Qualitätsbewertungsparametern der Beispieldaten einer Vielzahl klassifizierter Szenarien basierend auf Energieratios der physiologischen Daten, die verschiedenen Szenarien entsprechen; und

Bewerten (305) der Qualität eines von der Detektionsvorrichtung (200) gesammelten physiologischen Detektionssignals basierend auf den Qualitätsbewertungsparametern; **dadurch gekennzeichnet, dass** das Berechnen (S303) des Energieratios der physiologischen Daten basierend auf den physiologischen Daten und den PPG-Daten umfasst:

Separates Berechnen, basierend auf den physiologischen Daten und den PPG-Daten, einer Vielzahl von Energieratios der Frequenzen, die einer Vielzahl von physiologischen Daten entsprechen; und

Berechnen eines Durchschnittswerts der Vielzahl von Energieratios der Frequenzen, die einer Vielzahl von physiologischen Daten entsprechen, und Verwenden des Durchschnittswerts als Energieratio der physiologischen Daten.

2. Das Verfahren zur Bewertung der Signalqualität physiologischer Detektion gemäß Anspruch 1, wobei das Erhalten (S301) des von der Detektionsvorrichtung (200) in verschiedenen Szenarien gesammelten Beispieldatensatzes umfasst:

Festlegen (S3011) einer Vielzahl von voreingestellten Szenarien basierend auf einem Label;

Verbinden (S3012) der Detektionsvorrichtung (200) mit einem Datensammlungsendpunkt;

Sammeln (S3013) von Daten durch die Detektionsvorrichtung (200) basierend auf einem einzelnen voreingestellten Szenario, um ein einzelnes Stück Beispieldaten zu erzeugen; und

Ausgeben (S3014) eines Mehrszenario-Beispieldatensatzes basierend auf den Beispieldaten der Vielzahl von voreingestellten Szenarien.

3. Das Verfahren zur Bewertung der Signalqualität physiologischer Detektion gemäß Anspruch 1, wobei das separate Berechnen, basierend auf den physiologischen Daten und den PPG-Daten, der Vielzahl von Energieratios der Frequenzen, die einer Vielzahl von physiologischen Daten entsprechen, umfasst:

Durchführen (S3031) einer Kurzzeit-Fourier-Transformation der PPG-Daten, um die Leistungsdichtedaten der PPG-Daten zu erhalten, wobei der Fenstertyp der Kurzzeit-Fourier-Transformation ein Hamming-Fenster ist;

Bestimmen (S3033) von Zeitpunkten, die den Mittelpunkten einer Vielzahl von Hamming-Fenstern entsprechen, und Erfassen von physiologischen Daten, die jedem Zeitpunkt entsprechen;

Umwandeln (S3034) der physiologischen Daten in einen Frequenzbereich;

Berechnen (S3035) eines Energieratios, das dem Frequenzbereich entspricht, basierend auf den Leistungsdichtedaten; und

Ausgeben (S3036) einer Energieratioliste der Vielzahl von physiologischen Daten im Probendatensatz basierend auf den Energieratios, die einer Vielzahl von Frequenzbereichen entsprechen.

4. Das Verfahren zur Bewertung der Signalqualität der physiologischen Detektion gemäß Anspruch 3, wobei das Berechnen (S3035) des Energieratios, das dem Frequenzbereich entspricht, basierend auf den Leistungsdichtedaten Folgendes umfasst:
Teilen der Summe der Energiewerte, die einer Vielzahl von Frequenzwerten im Frequenzbereich entsprechen, durch die Summe der Energiewerte, die allen Frequenzwerten entsprechen, um das Energieratio zu erhalten, das dem Frequenzbereich entspricht.

5. Das Verfahren zur Bewertung der Signalqualität der physiologischen Detektion gemäß Anspruch 3, wobei das separate Berechnen der Vielzahl von Energieratios der Frequenzen, die der Vielzahl von physiologischen Daten entsprechen, basierend auf den physiologischen Daten und den PPG-Daten Folgendes umfasst:
Durchführen einer Interpolationsverarbeitung der Leistungsdichtedaten in einer zeitdomänen Nullfüllung oder einer frequenzdomänen Auffüllmethode.

6. Das Verfahren zur Bewertung der Signalqualität der physiologischen Detektion gemäß Anspruch 3, wobei das separate Berechnen der Vielzahl von Energieratios der Frequenzen, die der Vielzahl von physiologischen Daten entsprechen, basierend auf den physiologischen Daten und den PPG-Daten Folgendes umfasst:
Löschen von abnormalen Daten in der Energieratioliste der Vielzahl von physiologischen Daten; wobei das Löschen von abnormalen Daten in der Energieratioliste der Vielzahl von physiologischen Daten Folgendes umfasst:

Berechnen eines oberen Quartils Q1E und eines unteren Quartils Q3E in der Energieratioliste;

Berechnen einer oberen Schwelle U1 und einer unteren Schwelle U2 der abnormalen Daten;

Bestimmen, dass Daten, die in der Energieratioliste enthalten sind und die kleiner oder gleich der unteren Schwelle oder größer oder gleich der oberen Schwelle sind, die abnormalen Daten sind; und

Ausgeben einer Energieratioliste, aus der die abnormalen Daten gelöscht wurden.

7. Das Verfahren zur Bewertung der Signalqualität der physiologischen Erkennung gemäß Anspruch 1, wobei die Qualitätsbewertungsparameter ein unteres Quartil, ein Median und ein oberes Quartil der Energieraten physiologischer Daten in einer Vielzahl von Szenarien in den klassifizierten Szenarien umfassen; wobei die separate Berechnung der Qualitätsbewertungsparameter von Probendaten einer Vielzahl klassifizierter Szenarien basierend auf Energieraten der physiologischen Daten, die verschiedenen Szenarien entsprechen, umfasst:

Klassifizieren (S3041) der verschiedenen Szenarien, um die Vielzahl klassifizierter Szenarien zu bestimmen;

Erhalten (S3041), basierend auf einem Analyseobjekt, einer Vielzahl von Energieraten physiologischer Daten klassifizierter Szenarien, die jeweils mindestens zwei Bewertungsdimensionen entsprechen; und

Berechnen (S3043) eines unteren Quartils, eines Medians und eines oberen Quartils der Energieraten physiologischer Daten klassifizierter Szenarien, die jeweils mindestens zwei Bewertungsdimensionen entsprechen.

8. Das Verfahren zur Bewertung der Signalqualität der physiologischen Erkennung gemäß Anspruch 7, wobei die Bewertung der Qualität des von der Erkennungsvorrichtung (200) erfassten physiologischen Erkennungssignals basierend auf den Qualitätsbewertungsparametern umfasst:
Bestimmen, basierend auf einem Zunahme- oder Abnahmeratio eines Qualitätsbewertungsparameters eines klassifizierten Szenarios, das einer ersten Bewertungsdimension entspricht, zu einem Qualitätsbewertungsparameter eines klassifizierten Szenarios, das einer zweiten Bewertungsdimension entspricht, ob die Qualität des von der Erkennungsvorrichtung (200) erfassten physiologischen Erkennungssignals verbessert oder verschlechtert wird.

9. Das Verfahren zur Bewertung der Signalqualität der physiologischen Erkennung gemäß Anspruch 8, wobei das Bestimmen, basierend auf dem Zunahme- oder Abnahmeratio des Qualitätsbewertungsparameters des klassifizierten Szenarios, das der ersten Bewertungsdimension entspricht, zu dem Qualitätsbewertungsparameter des klassifizierten Szenarios, das der zweiten Bewertungsdimension entspricht, ob die Qualität des von der Erkennungsvorrichtung (200) erfassten physiologischen Erkennungssignals verbessert oder verschlechtert wird, umfasst:

Berechnen eines unteren Quartil-Zunahmeratios, eines Median-Zunahmeratios und eines oberen Quartil-Zunahmeratios einer Vielzahl von Energieraten physiologischer Daten des klassifizierten Szenarios, das der ersten Bewertungsdimension entspricht, zu einer Vielzahl von Energieraten physiologischer Daten des klassifizierten Szenarios, das der zweiten Bewertungsdimension entspricht; und
Bestimmen, basierend auf dem unteren Quartil-Zunahmeratio, dem Median-Zunahmeratio und dem oberen Quartil-Zunahmeratio, ob die Qualität des von der Erkennungsvorrichtung (200) erfassten physiologischen Erkennungssignals verbessert oder verschlechtert wird.

10. Das Verfahren zur Bewertung der Qualität von physiologischen Erkennungssignalen gemäß Anspruch 9, wobei das Bestimmen, basierend auf dem unteren Quartilszuwachsverhältnis, dem Medianzuwachsverhältnis und dem oberen Quartilszuwachsverhältnis, ob die Qualität des von der Erkennungsvorrichtung (200) erfassten physiologischen Erkennungssignals verbessert oder verschlechtert ist, umfasst:

Bestimmen, ob das untere Quartilszuwachsverhältnis, das Medianzuwachsverhältnis und das obere Quartilszuwachsverhältnis größer als 0 sind; und
Falls festgestellt wird, dass das untere Quartilszuwachsverhältnis, das Medianzuwachsverhältnis und das obere Quartilszuwachsverhältnis größer als 0 sind, wird festgestellt, dass die Qualität eines von der Erkennungsvorrichtung (200) erfassten physiologischen Erkennungssignals in der ersten Bewertungsdimension im Vergleich zur zweiten Bewertungsdimension verbessert ist;
Falls festgestellt wird, dass das untere Quartilszuwachsverhältnis, das Medianzuwachsverhältnis und das obere Quartilszuwachsverhältnis alle gleich 0 sind, wird festgestellt, dass die Qualität eines von der Erkennungsvorrichtung (200) erfassten physiologischen Erkennungssignals in der ersten Bewertungsdimension im Vergleich zur zweiten Bewertungsdimension unverändert bleibt; oder
Falls festgestellt wird, dass eines der unteren Quartilszuwachsverhältnisse, das Medianzuwachsverhältnis oder das obere Quartilszuwachsverhältnis kleiner als 0 ist, wird festgestellt, dass die Qualität eines von der Erkennungsvorrichtung (200) erfassten physiologischen Erkennungssignals in der ersten Bewertungsdimension im Vergleich zur zweiten Bewertungsdimension verschlechtert ist.

11. Das Verfahren zur Bewertung der Qualität von physiologischen Erkennungssignalen gemäß Anspruch 9, wobei das Bestimmen, basierend auf dem unteren Quartilszuwachsverhältnis, dem Medianzuwachsverhältnis und dem oberen Quartilszuwachsverhältnis, ob die Qualität des von der Erkennungsvorrichtung (200) erfassten physiologischen Erkennungssignals verbessert oder verschlechtert ist, umfasst:

Berechnung eines Durchschnittswerts des unteren Quartilszuwachsverhältnisses, des Medianzuwachsverhältnisses und des oberen Quartilszuwachsverhältnisses;
Bestimmen, ob der Durchschnittswert größer als 0 ist; und
Falls festgestellt wird, dass der Durchschnittswert größer als 0 ist, wird festgestellt, dass die Qualität eines von der Erkennungsvorrichtung (200) erfassten physiologischen Erkennungssignals in der ersten Bewertungsdimension im Vergleich zur zweiten Bewertungsdimension verbessert ist;
Falls festgestellt wird, dass der Durchschnittswert gleich 0 ist, wird festgestellt, dass die Qualität eines von der Erkennungsvorrichtung (200) erfassten physiologischen Erkennungssignals in der ersten Bewertungsdimension im Vergleich zur zweiten Bewertungsdimension unverändert bleibt; oder
Wenn festgestellt wird, dass der Durchschnittswert kleiner als 0 ist, wird festgestellt, dass die Qualität eines physiologischen Erkennungssignals, das von der Erkennungsvorrichtung (200) in der ersten Bewertungsdimension erfasst wurde, im Vergleich zu der in der zweiten Bewertungsdimension verschlechtert ist.

**12.** Das Verfahren zur Bewertung der Qualität physiologischer Erkennungssignale gemäß Anspruch 9, wobei die Bestimmung, basierend auf dem Anstieg des unteren Quartils, dem Anstieg des Medians und dem Anstieg des oberen Quartils, ob die Qualität des von der Erkennungsvorrichtung (200) erfassten physiologischen Erkennungssignals verbessert oder verschlechtert ist, umfasst:

Separates Festlegen von Gewichtswerten für den Anstieg des unteren Quartils, den Anstieg des Medians und den Anstieg des oberen Quartils;

Berechnung einer Summe des Anstiegs des unteren Quartils, des Anstiegs des Medians und des Anstiegs des oberen Quartils basierend auf den Gewichtswerten des Anstiegs des unteren Quartils, des Anstiegs des Medians und des Anstiegs des oberen Quartils;

Bestimmung, ob die Summe des Anstiegs des unteren Quartils, des Anstiegs des Medians und des Anstiegs des oberen Quartils größer als 0 ist; und

Wenn festgestellt wird, dass die Summe des Anstiegs des unteren Quartils, des Anstiegs des Medians und des Anstiegs des oberen Quartils größer als 0 ist, wird festgestellt, dass die Qualität eines physiologischen Erkennungssignals, das von der Erkennungsvorrichtung (200) in der ersten Bewertungsdimension erfasst wurde, im Vergleich zu der in der zweiten Bewertungsdimension verbessert ist;

Wenn festgestellt wird, dass die Summe des Anstiegs des unteren Quartils, des Anstiegs des Medians und des Anstiegs des oberen Quartils gleich 0 ist, wird festgestellt, dass die Qualität eines physiologischen Erkennungssignals, das von der Erkennungsvorrichtung (200) in der ersten Bewertungsdimension erfasst wurde, im Vergleich zu der in der zweiten Bewertungsdimension unverändert bleibt; oder

Wenn festgestellt wird, dass die Summe des Anstiegs des unteren Quartils, des Anstiegs des Medians und des Anstiegs des oberen Quartils kleiner als 0 ist, wird festgestellt, dass die Qualität eines physiologischen Erkennungssignals, das von der Erkennungsvorrichtung (200) in der ersten Bewertungsdimension erfasst wurde, im Vergleich zu der in der zweiten Bewertungsdimension verschlechtert ist.

**13.** Das Verfahren zur Bewertung der Qualität physiologischer Erkennungssignale gemäß Anspruch 1, wobei das Verfahren ferner umfasst:

Festlegen einer oberen und einer unteren Schwelle einer Bandpassfrequenz unter Verwendung eines Bandpassfilters n-ter Ordnung; und

Eingabe der PPG-Daten in den Bandpassfilter und Herausfiltern von niederfrequentem und/oder hochfrequentem Rauschen in den PPG-Daten basierend auf der oberen und der unteren Schwelle unter Verwendung des Bandpassfilters.

**14.** Ein elektronisches Gerät (100), wobei das elektronische Gerät (100) einen Speicher (121) und einen Prozessor (110) umfasst;

der Speicher (121) ist konfiguriert, Programmierbefehle zu speichern; und

der Prozessor (110) ist konfiguriert, die im Speicher (121) gespeicherten Programmierbefehle zu lesen und auszuführen, und wenn die Programmierbefehle vom Prozessor (110) ausgeführt werden, wird das elektronische Gerät (100) befähigt, das Verfahren zur Bewertung der Signalqualität der physiologischen Detektion gemäß einem der Ansprüche 1 bis 13 durchzuführen.

**Revendications**

**1.** Une méthode d'évaluation de la qualité du signal de détection physiologique, la méthode comprenant :

obtenir (S301) un ensemble de données d'échantillon collectées par un dispositif de détection (200) dans différents scénarios ;

sélectionner (S302) des données d'échantillon d'un scénario à partir de l'ensemble de données d'échantillon, et diviser les données d'échantillon en données physiologiques et données PPG ;

calculer (S303) un ratio d'énergie des données physiologiques basé sur les données physiologiques et les données PPG ;

calculer séparément (S304) des paramètres d'évaluation de qualité des données d'échantillon d'une pluralité de scénarios classifiés basés sur les ratios d'énergie des données physiologiques correspondant à différents scénarios ; et

évaluer (305), sur la base des paramètres d'évaluation de qualité, la qualité d'un signal de détection physiologique collecté par le dispositif de détection (200) ; **caractérisé en ce que** le calcul (S303) du ratio d'énergie des données physiologiques basé sur les données physiologiques et les données PPG comprend :

calculer séparément, sur la base des données physiologiques et des données PPG, une pluralité de ratios d'énergie des fréquences correspondant à une pluralité de données physiologiques ; et
calculer une valeur moyenne de la pluralité de ratios d'énergie des fréquences correspondant à la pluralité de données physiologiques, et utiliser la valeur moyenne comme le ratio d'énergie des données physiologiques.

2. La méthode d'évaluation de la qualité du signal de détection physiologique selon la revendication 1, où l'obtention (S301) de l'ensemble de données d'échantillon collectées par le dispositif de détection (200) dans différents scénarios comprend :

définir (S3011) une pluralité de scénarios prédéfinis sur la base d'une étiquette ;
connecter (S3012) le dispositif de détection (200) à une extrémité de collecte de données ;
collecter (S3013), par le dispositif de détection (200), des données basées sur un seul scénario prédéfini pour générer une seule donnée d'échantillon ; et
produire (S3014) un ensemble de données d'échantillon multi-scénarios basé sur les données d'échantillon de la pluralité de scénarios prédéfinis.

3. La méthode d'évaluation de la qualité du signal de détection physiologique selon la revendication 1, où le calcul séparé, sur la base des données physiologiques et des données PPG, de la pluralité de ratios d'énergie des fréquences correspondant à la pluralité de données physiologiques comprend :

effectuer (S3031) une transformée de Fourier à court terme sur les données PPG pour obtenir des données de densité spectrale de puissance des données PPG, où le type de fenêtre de la transformée de Fourier à court terme est une fenêtre de Hamming ;
déterminer (S3033) des points temporels correspondant aux emplacements centraux d'une pluralité de fenêtres de Hamming, et obtenir des données physiologiques correspondant à chaque point temporel ;
convertir (S3034) les données physiologiques en une plage de fréquences ;
calculer (S3035), sur la base des données de densité spectrale de puissance, un ratio d'énergie correspondant à la plage de fréquences ; et
produire (S3036) une liste de ratios d'énergie des multiples données physiologiques dans les données d'échantillon sur la base des ratios d'énergie correspondant à plusieurs plages de fréquences.

4. Le procédé d'évaluation de la qualité du signal de détection physiologique selon la revendication 3, dans lequel le calcul (S3035), sur la base des données de densité spectrale de puissance, du ratio d'énergie correspondant à la plage de fréquences comprend :
diviser une somme de valeurs d'énergie correspondant à plusieurs valeurs de fréquence dans la plage de fréquences par une somme de valeurs d'énergie correspondant à toutes les valeurs de fréquence pour obtenir le ratio d'énergie correspondant à la plage de fréquences.

5. Le procédé d'évaluation de la qualité du signal de détection physiologique selon la revendication 3, dans lequel le calcul séparé, sur la base des données physiologiques et des données PPG, des multiples ratios d'énergie des fréquences correspondant aux multiples données physiologiques comprend en outre :
effectuer un traitement d'interpolation sur les données de densité spectrale de puissance de manière à remplir les zéros dans le domaine temporel ou à ajouter des données dans le domaine fréquentiel.

6. Le procédé d'évaluation de la qualité du signal de détection physiologique selon la revendication 3, dans lequel le calcul séparé, sur la base des données physiologiques et des données PPG, des multiples ratios d'énergie des fréquences correspondant aux multiples données physiologiques comprend en outre :
supprimer les données anormales dans la liste des ratios d'énergie des multiples données physiologiques ; dans lequel la suppression des données anormales dans la liste des ratios d'énergie des multiples données physiologiques comprend :

calculer un quartile supérieur Q1E et un quartile inférieur Q3E dans la liste des ratios d'énergie ;
calculer un seuil supérieur U1 et un seuil inférieur U2 des données anormales ;
déterminer que les données qui figurent dans la liste des ratios d'énergie et qui sont inférieures ou égales au seuil inférieur ou supérieures ou égales au seuil supérieur sont les données anormales ; et

produire une liste de ratios d'énergie à partir de laquelle les données anormales sont supprimées.

7. Le procédé d'évaluation de la qualité du signal de détection physiologique selon la revendication 1, dans lequel les paramètres d'évaluation de la qualité comprennent un quartile inférieur, une médiane et un quartile supérieur des ratios d'énergie des données physiologiques de plusieurs scénarios dans les scénarios classifiés ; dans lequel le calcul séparé des paramètres d'évaluation de la qualité des données d'échantillon de plusieurs scénarios classifiés basé sur les ratios d'énergie des données physiologiques correspondant à différents scénarios comprend :

   classer (S3041) les différents scénarios pour déterminer les multiples scénarios classifiés ;
   obtenir (S3041), sur la base d'un objet d'analyse, plusieurs ratios d'énergie des données physiologiques des scénarios classifiés correspondant séparément à au moins deux dimensions d'évaluation ; et
   calculer (S3043) un quartile inférieur, une médiane et un quartile supérieur des ratios d'énergie des données physiologiques des scénarios classifiés correspondant séparément aux au moins deux dimensions d'évaluation.

8. Le procédé d'évaluation de la qualité du signal de détection physiologique selon la revendication 7, dans lequel l'évaluation, basée sur les paramètres d'évaluation de la qualité, de la qualité du signal de détection physiologique collecté par le dispositif de détection (200) comprend :
   déterminer, sur la base d'un ratio d'augmentation ou de diminution d'un paramètre d'évaluation de la qualité d'un scénario classifié correspondant à une première dimension d'évaluation par rapport à un paramètre d'évaluation de la qualité d'un scénario classifié correspondant à une deuxième dimension d'évaluation, si la qualité du signal de détection physiologique collecté par le dispositif de détection (200) est améliorée ou dégradée.

9. Le procédé d'évaluation de la qualité du signal de détection physiologique selon la revendication 8, dans lequel la détermination, sur la base du ratio d'augmentation ou de diminution du paramètre d'évaluation de la qualité du scénario classifié correspondant à la première dimension d'évaluation par rapport au paramètre d'évaluation de la qualité du scénario classifié correspondant à la deuxième dimension d'évaluation, si la qualité du signal de détection physiologique collecté par le dispositif de détection (200) est améliorée ou dégradée comprend :

   calculer un ratio d'augmentation du quartile inférieur, un ratio d'augmentation de la médiane et un ratio d'augmentation du quartile supérieur de plusieurs ratios d'énergie des données physiologiques du scénario classifié correspondant à la première dimension d'évaluation par rapport à plusieurs ratios d'énergie des données physiologiques du scénario classifié correspondant à la deuxième dimension d'évaluation ; et
   déterminer, sur la base du ratio d'augmentation du quartile inférieur, du ratio d'augmentation de la médiane et du ratio d'augmentation du quartile supérieur, si la qualité du signal de détection physiologique collecté par le dispositif de détection (200) est améliorée ou dégradée.

10. La méthode d'évaluation de la qualité du signal de détection physiologique selon la revendication 9, dans laquelle la détermination, basée sur le ratio d'augmentation du quartile inférieur, le ratio d'augmentation médian et le ratio d'augmentation du quartile supérieur, si la qualité du signal de détection physiologique collecté par le dispositif de détection (200) est améliorée ou dégradée, comprend :

   déterminer si le ratio d'augmentation du quartile inférieur, le ratio d'augmentation médian et le ratio d'augmentation du quartile supérieur sont supérieurs à 0 ; et
   s'il est déterminé que le ratio d'augmentation du quartile inférieur, le ratio d'augmentation médian et le ratio d'augmentation du quartile supérieur sont supérieurs à 0, déterminer que la qualité d'un signal de détection physiologique collecté par le dispositif de détection (200) dans la première dimension d'évaluation est améliorée par rapport à celle dans la deuxième dimension d'évaluation ;
   s'il est déterminé que le ratio d'augmentation du quartile inférieur, le ratio d'augmentation médian et le ratio d'augmentation du quartile supérieur sont tous égaux à 0, déterminer que la qualité d'un signal de détection physiologique collecté par le dispositif de détection (200) dans la première dimension d'évaluation reste inchangée par rapport à celle dans la deuxième dimension d'évaluation ; ou
   s'il est déterminé que l'un quelconque des ratios d'augmentation du quartile inférieur, médian ou supérieur est inférieur à 0, déterminer que la qualité d'un signal de détection physiologique collecté par le dispositif de détection (200) dans la première dimension d'évaluation est dégradée par rapport à celle dans la deuxième dimension d'évaluation.

11. La méthode d'évaluation de la qualité du signal de détection physiologique selon la revendication 9, dans laquelle la détermination, basée sur le ratio d'augmentation du quartile inférieur, le ratio d'augmentation médian et le ratio d'augmentation du quartile supérieur, si la qualité du signal de détection physiologique collecté par le dispositif de détection (200) est améliorée ou dégradée, comprend :

calculer une valeur moyenne des ratios d'augmentation du quartile inférieur, médian et supérieur ;
déterminer si la valeur moyenne est supérieure à o ; et
s'il est déterminé que la valeur moyenne est supérieure à 0, déterminer que la qualité d'un signal de détection physiologique collecté par le dispositif de détection (200) dans la première dimension d'évaluation est améliorée par rapport à celle dans la deuxième dimension d'évaluation ;
s'il est déterminé que la valeur moyenne est égale à 0, déterminer que la qualité d'un signal de détection physiologique collecté par le dispositif de détection (200) dans la première dimension d'évaluation reste inchangée par rapport à celle dans la deuxième dimension d'évaluation ; ou
s'il est déterminé que la valeur moyenne est inférieure à 0, déterminant que la qualité d'un signal de détection physiologique collecté par le dispositif de détection (200) dans la première dimension d'évaluation est dégradée par rapport à celle dans la deuxième dimension d'évaluation.

12. Le procédé d'évaluation de la qualité du signal de détection physiologique selon la revendication 9, dans lequel la détermination, basée sur le ratio d'augmentation du quartile inférieur, le ratio d'augmentation médian et le ratio d'augmentation du quartile supérieur, si la qualité du signal de détection physiologique collecté par le dispositif de détection (200) est améliorée ou dégradée comprend :

définir séparément les valeurs de poids du ratio d'augmentation du quartile inférieur, du ratio d'augmentation médian et du ratio d'augmentation du quartile supérieur ;
calculer une somme des ratios d'augmentation du quartile inférieur, du ratio d'augmentation médian et du ratio d'augmentation du quartile supérieur en fonction des valeurs de poids des ratios d'augmentation du quartile inférieur, du ratio d'augmentation médian et du ratio d'augmentation du quartile supérieur ;
déterminer si la somme des ratios d'augmentation du quartile inférieur, du ratio d'augmentation médian et du ratio d'augmentation du quartile supérieur est supérieure à 0 ; et
s'il est déterminé que la somme des ratios d'augmentation du quartile inférieur, du ratio d'augmentation médian et du ratio d'augmentation du quartile supérieur est supérieure à 0, déterminant que la qualité d'un signal de détection physiologique collecté par le dispositif de détection (200) dans la première dimension d'évaluation est améliorée par rapport à celle dans la deuxième dimension d'évaluation ;
s'il est déterminé que la somme des ratios d'augmentation du quartile inférieur, du ratio d'augmentation médian et du ratio d'augmentation du quartile supérieur est égale à 0, déterminant que la qualité d'un signal de détection physiologique collecté par le dispositif de détection (200) dans la première dimension d'évaluation reste inchangée par rapport à celle dans la deuxième dimension d'évaluation ; ou
s'il est déterminé que la somme des ratios d'augmentation du quartile inférieur, du ratio d'augmentation médian et du ratio d'augmentation du quartile supérieur est inférieure à 0, déterminant que la qualité d'un signal de détection physiologique collecté par le dispositif de détection (200) dans la première dimension d'évaluation est dégradée par rapport à celle dans la deuxième dimension d'évaluation.

13. Le procédé d'évaluation de la qualité du signal de détection physiologique selon la revendication 1, dans lequel le procédé comprend en outre:

définir un seuil supérieur et un seuil inférieur d'une fréquence de bande passante à l'aide d'un filtre passe-bande d'ordre f ; et
entrer les données PPG dans le filtre passe-bande et filtrer les bruits de basse fréquence et/ou de haute fréquence dans les données PPG en fonction du seuil supérieur et du seuil inférieur à l'aide du filtre passe-bande.

14. Un dispositif électronique (100), dans lequel le dispositif électronique (100) comprend une mémoire (121) et un processeur (110) ;

la mémoire (121) est configurée pour stocker des instructions de programme ; et
le processeur (110) est configuré pour lire et exécuter les instructions de programme stockées dans la mémoire (121), et lorsque les instructions de programme sont exécutées par le processeur (110), le dispositif électronique (100) est activé pour effectuer la méthode d'évaluation de la qualité du signal de détection physiologique selon l'une quelconque des revendications 1 à 13.

FIG. 1A

FIG. 1B

FIG. 1C

FIG. 2

S301

Obtain a sample data set of a detection device in different scenarios

S302

Select sample data of a scenario from the sample data set, and divide the sample data into physiological data and PPG data

S303

Calculate an energy ratio of the physiological data in the sample data based on the physiological data and the PPG data

S304

Separately calculate quality evaluation parameters of sample data of a plurality of classified scenarios based on energy ratios of the physiological data in different scenarios

S305

Evaluate, based on the quality evaluation parameters, quality of a PPG signal collected by the detection device

FIG. 3

S3011

Set a plurality of preset scenarios based on a label

S3012

Connect a medical device to a data collection end

S3013

Collect data based on a single scenario to generate a single piece of sample data

S3014

Output a multi-scenario sample data set based on sample data of each scenario

FIG. 4

S3031

Perform short-time Fourier transform on PPG data to obtain power spectral density data of the data

S3032

Perform interpolation processing on the power spectral density data

S3033

Determine time points corresponding to center locations of a plurality of Hamming windows, and obtain physiological data corresponding to each time point

S3034

Convert the obtained physiological data into a frequency range

S3035

Calculate, based on the power spectral density data, an energy ratio corresponding to the frequency range

S3036

Output an energy ratio list of a plurality of pieces of physiological data in the sample data based on energy ratios corresponding to a plurality of frequency ranges

FIG. 5

FIG. 6A

FIG. 6B

FIG. 7

FIG. 8

FIG. 9

FIG. 10

S101

Obtain a sample data set of a detection device in different scenarios

S102

Select sample data of a scenario from the sample data set, and divide the sample data into physiological data and PPG data

S103

Perform upsampling processing on the PPG data in the sample data

S104

Calculate an energy ratio of the physiological data in the sample data based on the physiological data and the PPG data

S105

Separately calculate quality evaluation parameters of sample data of a plurality of classified scenarios based on energy ratios of the physiological data in different scenarios

S106

Evaluate, based on the quality evaluation parameters, quality of a PPG signal collected by the detection device

FIG. 11

S201

Obtain a sample data set of a detection device in different scenarios

S202

Select sample data of a scenario from the sample data set, and divide the sample data into physiological data and PPG data

S203

Perform upsampling processing on the PPG data in the sample data

S204

Filter out low-frequency/high-frequency noise in the PPG data

S205

Calculate an energy ratio of the physiological data in the sample data based on the physiological data and the PPG data

S206

Separately calculate quality evaluation parameters of sample data of a plurality of classified scenarios based on energy ratios of the physiological data in different scenarios

S207

Evaluate, based on the quality evaluation parameters, quality of a PPG signal collected by the detection device

FIG. 12

EP 4 282 323 B1

S1301

Multi-scenario
sample set

S1304

Heart rate label
data

S1302

Single sample

Yes

S1303

PPG data

S1305

Upsampling

S1306

Remove low-
frequency and
high-frequency
noise

S1307

Calculate an energy ratio
of ±5 BPM of a heart
rate per second of the
single sample

S1310

Whether
there is a remaining
sample that is not
analyzed

S1309

Calculate an energy ratio
of ±5 BPM of a heart
rate of the single sample

S1308

Delete an
abnormal frame
(second)

No

S1311

Statistical analysis
of all sample
results

S1312

Evaluation of an
iteration effect of
hardware

FIG. 13

Electronic device 100

FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 115700538 A

- US 2020337574 A1